# EUROPEAN PATENT APPLICATION

(11) **EP 4 413 916 A2**
(43) Date of publication of application: **14.08.2024**
(21) Application number: 24182193.3
(22) Date of filing: 10.07.2020
(51) Int. Cl.: A61B 3/14

(54) **OPHTHALMIC DEVICE AND METHOD FOR CONTROLLING SAME**

(30) Priority: 23.01.2020 JP 2020009456
(62) Divisional of application: 20915808.8
(71) Applicant: TOPCON CORPORATION, Tokyo 174-8580 (JP)
(72) Inventor: SUZUKI, Masaya, Tokyo, 174-8580 (JP); NAKAJIMA, Masashi, Tokyo, 174-8580 (JP); SHIMIZU, Hitoshi, Tokyo, 174-8580 (JP)
(74) Representative: Gagel, Roland

(57) **Abstract**

In a method of controlling an ophthalmic apparatus, the ophthalmic apparatus comprises:
a light source; an illumination optical system including a slit with a slit-shaped aperture capable of being arranged at a position substantially conjugate optically to a fundus of a subject's eye, and configured to generate slit-shaped illumination light using light from the light source; a first movement mechanism configured to move the slit in an optical axis direction of the illumination optical system; an optical scanner configured to deflect the illumination light to guide the illumination light to the fundus; an imaging optical system configured to guide returning light of the illumination light from the fundus to an image sensor; and a controller configured to control the image sensor using a rolling shutter method so as to acquire light receiving result of the returning light corresponding to an irradiated position of the illumination light on the fundus. The method comprises an acquisition step of acquiring a dioptric power of the subject's eye, and a first control step of controlling the first movement mechanism based on the dioptric power acquired in the acquisition step.

## Description

### [TECHNICAL FIELD]

The present invention relates to a method of controlling an ophthalmic apparatus.

### [BACKGROUND ART]

In recent years, screening tests have been performed using ophthalmic apparatuses. Such ophthalmic apparatuses are expected to be applied to self-examinations, and further downsizing and weight saving of the ophthalmic apparatuses are desired.

For example, Patent Document 1 and Patent Document 2 disclose an ophthalmic apparatus configured to pattern-illuminate a subject's eye using slit light and to detect returning light of the slit light using a CMOS (Complementary Metal Oxide Semiconductor) image sensor. This ophthalmic apparatus can acquire images of the subject's eye with a simple configuration, by adjusting the illumination pattern and the timing of light receiving timing using the CMOS image sensor.

### [PRIOR ART DOCUMENTS]

### [PATENT DOCUMENTS]

[Patent Document 1] US Patent No. 7831106
[Patent Document 2] US Patent No. 8237835

### [SUMMARY OF THE INVENTION]

### [PROBLEMS TO BE SOLVED BY THE INVENTION]

However, when the pupil of the subject's eye is a microcoria, it is known that the amount of light entering the eye decreases and the acquired image of the subject's eye (especially in the center) becomes darker. In addition, depending on the state of the subject's eye (dioptric power, etc.), image quality may be degraded.

The present invention has been made in view of such circumstances, and an object of the present invention is to provide a new technique for acquiring a high quality image of a subject's eye with a simple configuration, without being affected by the state of the subject's eye.

### [MEANS OF SOLVING THE PROBLEMS]

The first aspect of some embodiments is an ophthalmic apparatus, including: a light source; an illumination optical system configured to generate slit-shaped illumination light using light from the light source; an optical scanner configured to deflect the illumination light to guide the illumination light to a fundus of a subject's eye; an imaging optical system configured to guide returning light of the illumination light from the fundus to an image sensor; and a controller configured to control the image sensor using a rolling shutter method so as to acquire light receiving result of the returning light corresponding to an irradiated position of the illumination light on the fundus, wherein the illumination optical system includes: a slit with a slit-shaped aperture capable of being arranged at a position substantially conjugate optically to the fundus; an iris aperture arranged between the light source and the slit, and configured to be capable of being arranged at a position substantially conjugate optically to an iris of the subject's eye; and an optical element arranged between the light source and the iris aperture, and configured to deflect the light from the light source.

The second aspect of some embodiments is an ophthalmic apparatus, including: a light source; an illumination optical system including a slit with a slit-shaped aperture capable of being arranged at a position substantially conjugate optically to a fundus of a subject's eye, and configured to generate slit-shaped illumination light using light from the light source; a first movement mechanism configured to move the slit in an optical axis direction of the illumination optical system; an optical scanner configured to deflect the illumination light to guide the illumination light to the fundus; an imaging optical system configured to guide returning light of the illumination light from the fundus to an image sensor; and a controller configured to control the image sensor using a rolling shutter method so as to acquire light receiving result of the returning light corresponding to an irradiated position of the illumination light on the fundus, wherein the controller is configured to control the first movement mechanism based on a dioptric power of the subject's eye.

The third aspect of some embodiments, in the second aspect, further includes a second movement mechanism configured to change at least one of a position of the light source or an orientation of the light source, wherein the controller is configured to control the second movement mechanism according to the position of the slit moved by the first movement mechanism.

In the fourth aspect of some embodiments, in the third aspect, the illumination optical system includes an iris aperture arranged between the light source and the slit, and configured to be capable of being arranged at a position substantially conjugate optically to an iris of the subject's eye, and the controller is configured to control the second movement mechanism so that light having passed through the iris aperture passes through the aperture.

In the fifth aspect of some embodiments, in the fourth aspect, the illumination optical system includes a first relay lens system arranged between the optical scanner and the slit, and a back focal position of the first relay lens system is a position substantially conjugate optically to the iris.

In the sixth aspect of some embodiments, in the fifth aspect, the optical scanner is arranged at the back focal position or the vicinity of the back focal position.

The seventh aspect of some embodiments, in the fifth aspect or the sixth aspect, further includes: a light source for observation; an optical path coupling member arranged between the first relay lens system and the iris aperture, and configured to couple an optical path of light output from the light source and an optical path of light output from the light source for observation; and an iris aperture for observation arranged between the light source for observation source and the optical path coupling member.

The eighth aspect of some embodiments, in the fifth aspect or the sixth aspect, further includes: a second relay lens system arranged between the slit and the iris aperture, wherein the iris aperture is arranged at a front focal position of the second relay lens system or the vicinity of the front focal position of the second relay lens system.

In the ninth aspect of some embodiments, in the eighth aspect, at least one of a dioptric power of the first lens or a dioptric power of the second lens can be changed.

In the tenth aspect of some embodiments, in the ninth aspect, at least one of the dioptric power of the first lens or the dioptric power of the second lens can be changed according to a size of a light emitting surface of the light source.

The eleventh aspect of some embodiments, in any one of the eighth aspect to the tenth aspect, further includes : a light source for observation; an optical path coupling member arranged between the second relay lens system and the iris aperture, and configured to couple an optical path of light output from the light source and an optical path of light output from the light source for observation; and an iris aperture for observation arranged between the light source for observation and the optical path coupling member.

In the twelfth aspect of some embodiments, in any one of the fourth aspect to the eleventh aspect, the iris aperture has one or more apertures that the illumination light passes through so that luminous flux cross section of the illumination light and luminous flux cross section of returning light from the subject's eye are separated on a cornea of the subject's eye, an anterior surface of lens of the subject's eye, and a posterior surface of lens of the subject's eye.

In the thirteenth aspect of some embodiments, in the twelfth aspect, the iris aperture has two or more apertures, and the two or more apertures are formed in linear symmetry to a straight line, the straight line passing through an optical axis of the illumination optical system and extending in a direction corresponding to a longitudinal direction of the aperture formed in the slit.

In the fourteenth aspect of some embodiments, in the thirteenth aspect, the aperture has a circular segment shape, and a direction of a chord of the circular segment shape is approximately parallel to a direction corresponding to the longitudinal direction of the aperture formed in the slit.

In the fifteenth aspect of some embodiments, in any one of the fourth aspect to the fourteenth aspect, the illumination optical system includes an optical element arranged between the light source and the iris aperture, and configured to deflect light from the light source.

In the sixteenth aspect of some embodiments, in the first aspect or the fifteenth aspect, the optical element is configured to deflect the light from the light source so that a light amount distribution in a direction connecting the iris aperture and the aperture is maximized.

The seventeenth aspect of some embodiments, in the first aspect, the fifteenth aspect, or the sixteenth aspect, further includes a third movement mechanism configured to change at least one of a position of the optical element or an orientation of the optical element, wherein the controller is configured to control the third movement mechanism.

In the eighteenth aspect of some embodiments, in any one of the first aspect, the fifteenth aspect to the seventeenth aspect, wherein the optical element includes a prism, a microlens array, or a Fresnel lens.

The nineteenth aspect of some embodiments is a method of controlling an ophthalmic apparatus, the ophthalmic apparatus including: a light source; an illumination optical system including a slit with a slit-shaped aperture capable of being arranged at a position substantially conjugate optically to a fundus of a subject's eye, and configured to generate slit-shaped illumination light using light from the light source; a first movement mechanism configured to move the slit in an optical axis direction of the illumination optical system; an optical scanner configured to deflect the illumination light to guide the illumination light to the fundus; an imaging optical system configured to guide returning light of the illumination light from the fundus to an image sensor; and a controller configured to control the image sensor using a rolling shutter method so as to acquire light receiving result of the returning light corresponding to an irradiated position of the illumination light on the fundus. The method of controlling the ophthalmic apparatus includes: an acquisition step of acquiring a dioptric power of the subject's eye; and a first control step of controlling the first movement mechanism based on the dioptric power acquired in the acquisition step.

In the twentieth aspect of some embodiments, in the nineteenth aspect, the ophthalmic apparatus includes a second movement mechanism configured to change at least one of a position of the light source or an orientation of the light source. The method of controlling the ophthalmic apparatus further includes a second control step of controlling the second movement mechanism according to the position of the slit moved by the first movement mechanism.

In the twenty-first aspect of some embodiments, in the twentieth aspect, the illumination optical system includes an iris aperture arranged between the light source and the slit, and configured to be capable of being arranged at a position substantially conjugate optically to an iris of the subject's eye. In the method of controlling the ophthalmic apparatus, the second control step is performed to control the second movement mechanism so that light having passed through the iris aperture passes through the aperture.

In the twenty-second aspect of some embodiments, in the twenty-first aspect, the illumination optical system includes an optical element arranged between the light source and the iris aperture, and configured to deflect light from the light source, the ophthalmic apparatus includes a third movement mechanism configured to change at least one of a position of the optical element or an orientation of the optical element. The method of controlling the ophthalmic apparatus further includes a third control step of controlling the third movement mechanism.

In the twenty-third aspect of some embodiments, in the twenty-second aspect, the optical element includes a prism, a microlens array, or a Fresnel lens.

It should be noted that the configurations according to a plurality of aspects described above can be combined arbitrarily.

### [EFFECTS OF THE INVENTION]

According to the present invention, a new technique for acquiring a high quality image of a subject's eye with a simple configuration, without being affected by the state of the subject's eye, can be provided.

### [BRIEF EXPLANATION OF THE DRAWINGS]

[FIG. 1] FIG. 1 is a schematic diagram illustrating an example of a configuration of an optical system of an ophthalmic apparatus according to a first embodiment.
[FIG. 2] FIG. 2 is a schematic diagram illustrating an example of a configuration of a control system of the ophthalmic apparatus according to the first embodiment.
[FIG. 3] FIG. 3 is a schematic diagram illustrating an example of the configuration of the ophthalmic apparatus according to the first embodiment.
[FIG. 4] FIG. 4 is an explanatory diagram of an operation of the ophthalmic apparatus according to the first embodiment.
[FIG. 5] FIG. 5 is an explanatory diagram of an operation of the ophthalmic apparatus according to the first embodiment.
[FIG. 6] FIG. 6 is an explanatory diagram of an operation of the ophthalmic apparatus according to the first embodiment.
[FIG. 7] FIG. 7 is an explanatory diagram of an operation of the ophthalmic apparatus according to the first embodiment.
[FIG. 8] FIG. 8 is an explanatory diagram of an operation of the ophthalmic apparatus according to the first embodiment.
[FIG. 9] FIG. 9 is a flowchart illustrating an example of an operation of the ophthalmic apparatus according to the first embodiments.
[FIG. 10] FIG. 10 is a schematic diagram illustrating an example of a configuration of the ophthalmic apparatus according to a second embodiment.
[FIG. 11] FIG. 11 is a schematic diagram illustrating an example of a configuration of the ophthalmic apparatus according to a third embodiment.
[FIG. 12] FIG. 12 is an explanatory diagram of an example of the configuration of the ophthalmic apparatus according to the third embodiment.
[FIG. 13] FIG. 13 is an explanatory diagram of an example of the configuration of the ophthalmic apparatus according to the third embodiment.
[FIG. 14] FIG. 14 is a schematic diagram illustrating an example of a configuration of the ophthalmic apparatus according to a fourth embodiment.
[FIG. 15] FIG. 15 is a schematic diagram illustrating an example of the configuration of the ophthalmic apparatus according to the fourth embodiment.
[FIG. 16] FIG. 16 is a schematic diagram illustrating an example of a configuration of the ophthalmic apparatus according to a fifth embodiment.
[FIG. 17] FIG. 17 is a schematic diagram illustrating an example of a configuration of the ophthalmic apparatus according to a sixth embodiment.
[FIG. 18] FIG. 18 is an explanatory diagram of an example of the configuration of the ophthalmic apparatus according to the sixth embodiment.
[FIG. 19] FIG. 19 is a schematic diagram illustrating an example of a configuration of the ophthalmic apparatus according to a seventh embodiment.

### [MODES FOR CARRYING OUT THE INVENTION]

Referring now to the drawings, exemplary embodiments of an ophthalmic apparatus and a method of controlling the ophthalmic apparatus according to the present invention are described below. The contents of the document cited in the present specification can be appropriately incorporated as contents of the following embodiments.

An ophthalmic apparatus according to embodiments illuminates a predetermined site of a subject's eye while moving an irradiated position (irradiated range) of slit-shaped illumination light, and receives returning light from the predetermined site using an image sensor with a one-dimensional or two-dimensional array of light receiving elements. Light receiving result of the returning light is read out from the light receiving elements at light receiving position of the returning light corresponding to the irradiated position of the illumination light, in synchronization with the movement timing of the irradiated position of the illumination light. In some embodiments, the predetermined site is an anterior segment or a posterior segment. Examples of the anterior segment include a cornea, an iris, a crystalline lens, a ciliary body, and a ciliary zonule. Examples of the posterior segment include a vitreous body, and a fundus or the vicinity of the fundus (retina, choroid, sclera, etc.).

A method of controlling the ophthalmic apparatus according to the embodiments includes one or more steps for realizing the processing executed by a processor (computer) in the ophthalmic apparatus according to the embodiments. A program according to the embodiments causes the processor to execute each step of the method of controlling the ophthalmic apparatus according to the embodiments.

The term "processor" as used herein refers to a circuit such as, for example, a central processing unit (CPU), a graphics processing unit (GPU), an application specific integrated circuit (ASIC), and a programmable logic device (PLD). Examples of PLD include a simple programmable logic device (SPLD), a complex programmable logic device (CPLD), and a field programmable gate array (FPGA). The processor realizes, for example, the function according to the embodiments by reading out a computer program stored in a storage circuit or a storage device and executing the computer program.

Hereinafter, a case where the ophthalmic apparatus according to the embodiments acquires images of the fundus of the subject's eye mainly will be described.

### <First embodiment>

### [Configuration of optical system]

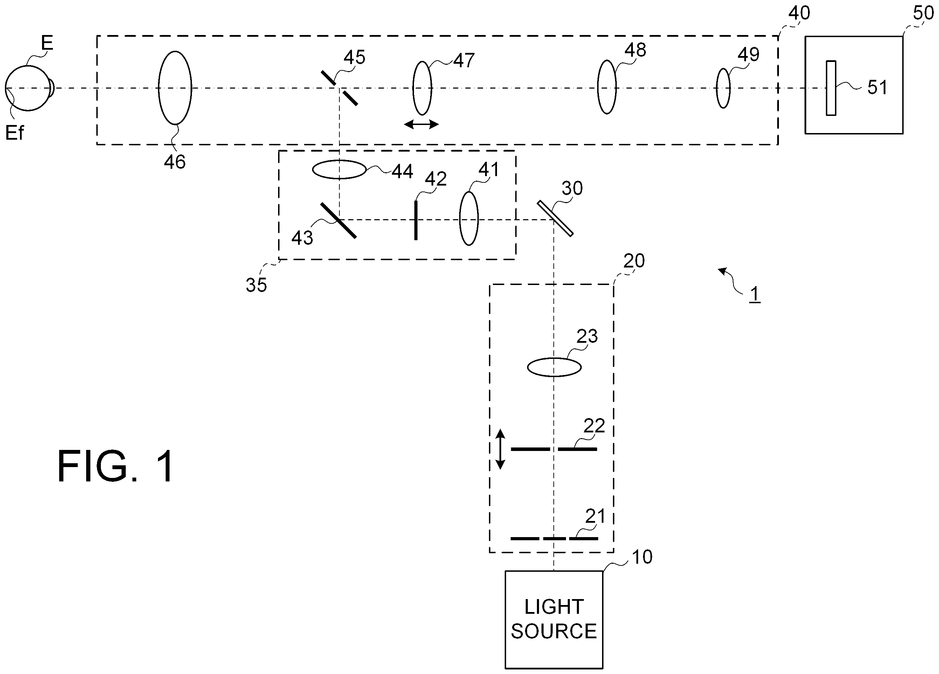

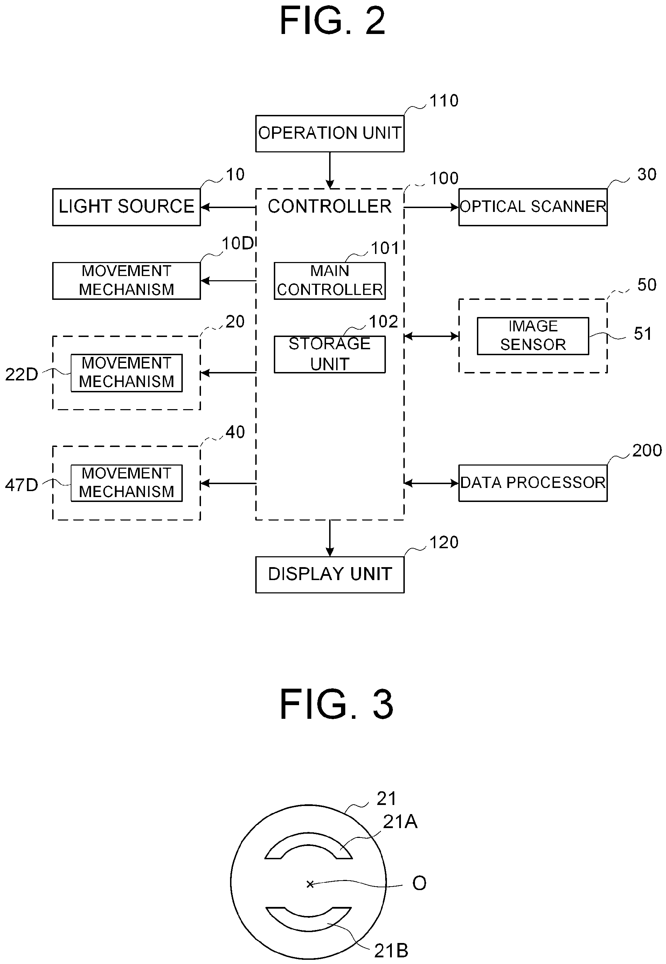

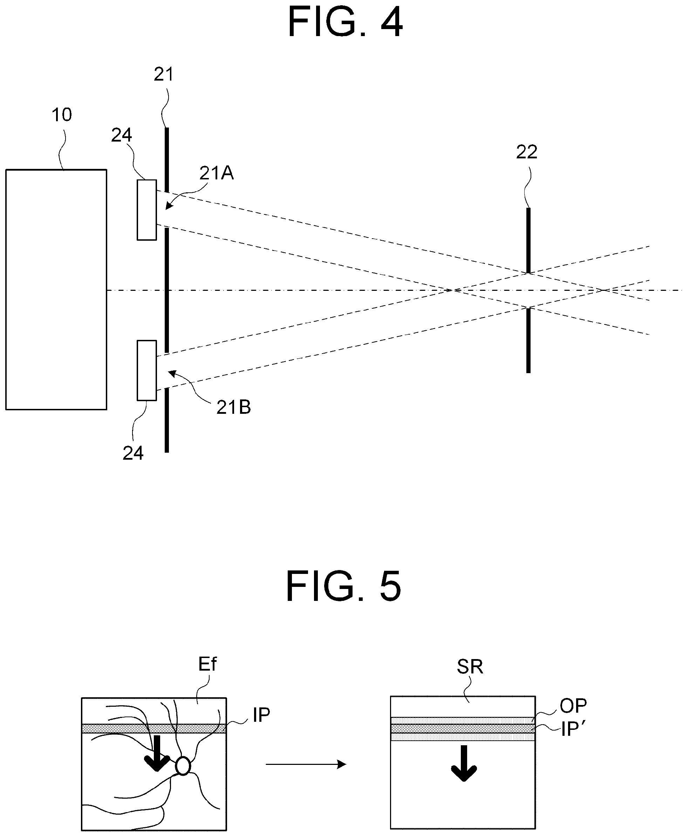

FIGs. 1 to 4 show block diagrams of examples of the configuration of the ophthalmic apparatus according to a first embodiment. FIG. 1 represents an example of the configuration of an optical system of the ophthalmic apparatus 1 according to the first embodiment. FIG. 2 representing a block diagram of an example of the configuration of the control system (processing system) of the ophthalmic apparatus 1 according to the first embodiment. FIG. 3 schematically represents an example of the configuration of an iris aperture 21 in FIG. 1 when viewed from a direction of an optical axis O. FIG. 4 represents an example of the configuration of the iris aperture 21 in FIG. 1 and a slit 22 in FIG. 1 when viewed from the side or top. In FIGs. 1 to 4, like parts are designated by like reference numerals as in repetitious description of such parts may not be provided.

The ophthalmic apparatus 1 includes a light source 10, an illumination optical system 20, an optical scanner 30, a projection optical system 35, and an imaging optical system 40, and an imaging device 50. In some embodiments, the illumination optical system 20 includes at least one of the light source 10, the optical scanner 30, or the projection optical system 35. In some embodiments, the imaging optical system 40 includes the imaging device 50. In some embodiments, the projection optical system 35 or the illumination optical system 20 includes the optical scanner 30.

### (Light source 10)

The light source 10 includes a visible light source that generates light in the visible region. For example, the light source 10 generates light having a central wavelength in the wavelength range of 420 nm to 700 nm. This type of light source 10 includes, for example, an LED (Light Emitting Diode), an LD (Laser Diode), a halogen lamp, or a xenon lamp. In some embodiments, the light source 10 includes a white light source or a light source capable of outputting light with each color component of RGB. In some embodiments, the light source 10 includes a light source capable of switching to output the light in infrared region or the light in visible region. The light source 10 is arranged at a position non-conjugate optically to each of a fundus Ef and the iris.

### (Illumination optical system 20)

The illumination optical system 20 generates slit-shaped illumination light using the light from the light source 10. The illumination optical system 20 guides the generated illumination light to the optical scanner 30.

The illumination optical system 20 includes the iris aperture 21, the slit 22, and a relay lens 23. The light from the light source 10 passes through the aperture(s) formed in the iris aperture 21, passes through the aperture formed in the slit 22, and is transmitted through the relay lens 23. The relay lens 23 includes one or more lenses. The light transmitted through the relay lens 23 is guided to the optical scanner 30.

### (Iris aperture 21)

The iris aperture 21 (specifically, aperture(s) described below) can be arranged at a position substantially conjugate optically to the iris (pupil) of a subject's eye E. In the iris aperture 21, one or more apertures are formed at position(s) away from the optical axis O. For example, as shown in FIG. 3, apertures 21A and 21B having a predetermined thickness along a circumferential direction centered with the optical axis O are formed in the iris aperture 21. The aperture(s) formed in the iris aperture 21 defines an incident position (incident shape) of the illumination light on the iris of the subject's eye E. For example, when the pupil center of the subject's eye E is arranged on the optical axis O, the illumination light can enter into the eye from positions deviated from the pupil center (specifically, point-symmetrical positions centered on the pupil center), by forming the apertures 21A and 21B as shown in FIG. 3.

As shown in FIG. 4, an optical element 24 is arranged between the light source 10 and the iris aperture 21. The optical element 24 can be arranged at a position substantially conjugate optically to the iris. The optical element 24 deflects the light from the light source. The optical element 24 deflects the light from the light source 10 so that the light amount distribution in a direction connecting the aperture 21A (or aperture 21B) formed in the iris aperture 21 and an aperture formed in the slit 22 is maximized. Examples of such optical element include a prism, a microlens array, or a Fresnel lens. In FIG. 4, the optical element 24 is provided for each aperture formed in the iris aperture 21. However, a single element may be configured to deflect the light passing through the apertures 21A and 21B formed in the iris aperture 21.

Further, the light amount distribution of the light passing through the aperture(s) formed in the iris aperture 21 can be changed by changing a relative position between the light source 10 and the aperture(s) formed in the iris aperture 21.

### (Slit 22)

The slit 22 (specifically, aperture(s) described below) can be arranged at a position substantially conjugate optically to the fundus Ef of the subject's eye E. For example, in the slit 22, the aperture is formed extending in a direction corresponding to a line direction (row direction) that is read out from the image sensor 51 described below using the rolling shutter method. The aperture formed in the slit 22 defines an irradiated pattern of the illumination light on the fundus Ef of the subject's eye E.

The slit 22 can be moved in the optical axis direction of the illumination optical system 20 using a movement mechanism (movement mechanism 22D described below). The movement mechanism moves the slit 22 in the optical axis direction, under the control from the controller 100 described below. For example, the controller 100 controls the movement mechanism in accordance with the state of the subject's eye E. This allows to move the position of the slit 22 in accordance with the state of the subject's eye E (specifically, the dioptric power or the shape of the fundus Ef).

In some embodiments, the slit 22 is configured so that at least one of the position of the aperture or the shape of the aperture can be changed in accordance with the state of the subject's eye E without being moved in the optical axis direction. The function of the slit 22 with this configuration is, for example, realized by a liquid crystal shutter.

The light from the light source 10 that has passed through the aperture(s) formed in the iris aperture 21 is output as the slit-shaped illumination light by passing through the aperture formed in the slit 22. The slit-shaped illumination light is transmitted through the relay lens 23, and is guided to the optical scanner 30.

### (Optical scanner 30)

The optical scanner 30 is placed at a position substantially conjugate optically to the iris of the subject's eye E. The optical scanner 30 deflects the slit-shaped illumination light transmitted through the relay lens 23 (slit-shaped light passing through the aperture formed in the slit 22). Specifically, the optical scanner 30 deflects the slit-shaped illumination light for sequentially illuminating a predetermined irradiated region of the fundus Ef to guide the illumination light to the projection optical system 35, while changing the deflection angle within a predetermined deflection angle range with the iris or the vicinity of the iris of the subject's eye E as a scan center position. The optical scanner 30 can deflect the illumination light one-dimensionally or two-dimensionally.

In case that the optical scanner 30 deflects the illumination light one-dimensionally, the optical scanner 30 includes a galvano scanner that deflects the illumination light within a predetermined deflection angle range with reference to a predetermined deflection direction. In case that the optical scanner 30 deflects the illumination light two-dimensionally, the optical scanner 30 includes a first galvano scanner and a second galvano scanner. The first galvano scanner deflects the illumination light so as to move the irradiated position of the illumination light in a horizontal direction orthogonal to the optical axis of the illumination optical system 20. The second galvano scanner deflects light deflected by the first galvano scanner so as to move the irradiated position of the illumination light in a vertical direction orthogonal to the optical axis of the illumination optical system 20. Examples of scan mode for moving the irradiated position of the illumination light using the optical scanner 30 include a horizontal scan, a vertical scan, a cross scan, a radial scan, a circle scan, a concentric scan, and a helical (spiral) scan.

### (Projection optical system 35)

The projection optical system 35 guides the illumination light deflected by the optical scanner 30 to the fundus Ef of the subject's eye E. In the embodiments, the projection optical system 35 guides the illumination light deflected by the optical scanner 30 through an optical path coupled with an optical path of the imaging optical system 40 by a perforated mirror 45 as the optical path coupling member described below.

The projection optical system 35 includes a relay lens 41, a black point plate 42, a reflective mirror 43, and a relay lens 44. Each of the relay lenses 41 and 44 includes one or more lenses.

### (Black point plate 42)

The black point plate 42 is arranged at a position substantially conjugate optically to a lens surface of an objective lens 46 or the vicinity of the lens surface of the objective lens 46. This prevents the reflected light from the lens surface of the objective lens 46 from being guided to the light source 10.

With such projection optical system 35, the illumination light deflected by the optical scanner 30 is transmitted through the relay lens 41, passes through the black point plate 42, is reflected by the reflective mirror 43 toward the perforated mirror 45.

### (Imaging optical system 40)

The imaging optical system 40 guides the illumination light that has been guided through the projection optical system 35 to the fundus Ef of the subject's eye E, and also guides the returning light of the illumination light from the fundus Ef to the imaging device 50.

In the imaging optical system 40, an optical path of the illumination light from the projection optical system 35 and an optical path of the returning light of the illumination light from the fundus Ef are coupled. By using the perforated mirror 45 as an optical path coupling member to couple these optical paths, it enables pupil division between the illumination light and the returning light of the illumination light.

The imaging optical system 40 includes the perforated mirror 45, the objective lens 46, a focusing lens 47, a relay lens 48, and an imaging lens 49. Each of relay lens 48 includes one or more lenses.

### (Perforated mirror 45)

In the perforated mirror 45, the hole is formed. The hole is arranged on the optical axis of the imaging optical system 40. The hole of the perforated mirror 45 is arranged at a position substantially conjugate optically to the iris of the subject's eye E. The perforated mirror 45 reflects the illumination light from the projection optical system 35 toward the objective lens 46, on the peripheral region of the hole. The perforated mirror 45 with this configuration functions as a photographic stop (diaphragm).

That is, the perforated mirror 45 is configured to couple the optical path of the illumination optical system 20 (projection optical system 35) and the optical path of the imaging optical system 40 arranged in a direction of the optical axis passing through the hole, and also to guide the illumination light reflected on the peripheral region of the hole to the fundus Ef.

### (Focusing lens 47)

The focusing lens 47 can be moved in an optical axis direction of the imaging optical system 40 using a movement mechanism (not shown). The movement mechanism moves the focusing lens 47 in the optical axis direction under the control from the controller 100 described below. This allows to image the returning light of the illumination light passing through the hole of the perforated mirror 45 on the light receiving surface of the image sensor 51 in the imaging device 50 in accordance with the state of the subject's eye E.

In the imaging optical system 40 with this configuration, the illumination light from the projection optical system 35 is reflected toward the objective lens 46 on the peripheral region of the hole formed in the perforated mirror 45. The illumination light reflected on the peripheral region of perforated mirror 45 is refracted by the objective lens 46, enters into the eye through the pupil of the subject's eye E, and illuminates the fundus Ef of the subject's eye E.

The returning light of the illumination light from the fundus Ef is refracted by the objective lens 46, passes through the hole of the perforated mirror 45, is transmitted through the focusing lens 47, is transmitted through the relay lens 48, and is imaged on the light receiving surface of the image sensor 51 in the imaging device 50 through the imaging lens 49.

### (Imaging device 50)

The imaging device 50 includes the image sensor 51 receiving the returning light of the illumination light that has been guided from the fundus Ef of the subject's eye E through the imaging optical system 40. The imaging device 50 can perform readout control of the light receiving result of the returning light under the control from the controller 100 described below.

### (Image sensor 51)

The image sensor 51 realizes the function as a pixelated photodetector. The light receiving surface (detecting surface, imaging surface) of the image sensor 51 can be arranged at a position substantially conjugate optically to the fundus Ef.

The light receiving result(s) obtained using the image sensor 51 is/are read out using a rolling shutter method under the control from the controller 100 described below.

The image sensor 51 with this configuration includes the CMOS image sensor. In this case, the image sensor 51 includes a plurality of pixels (light receiving elements). The plurality of pixels includes a plurality of pixel groups arranged in a column direction. Each of the plurality of pixel groups includes pixels arranged in a row direction. Specifically, the image sensor 51 includes a plurality of pixels arranged two-dimensionally, a plurality of vertical signal lines, and a horizontal signal line. Each pixel includes a photodiode (light receiving element), and a capacitor. The vertical signal lines are provided for each pixel group in the column direction (vertical direction) orthogonal to the row direction (horizontal direction). Each of the vertical signal lines is selectively electrically connected to the pixel group in which the electrical charge corresponding to the light receiving result is accumulated. The horizontal signal line is selectively electrically connected to the vertical signal lines. Each of the pixels accumulates the electrical charge corresponding to the light receiving result of the returning light. The accumulated electrical charge is read out sequentially for each pixel group in the row direction, for example. For example, for each line in the row direction, a voltage corresponding to the electrical charge accumulated in each pixel is supplied to the vertical signal line. The vertical signal lines are selectively electrically connected to the horizontal signal line. By performing readout operation for each line in the row direction described above sequentially in the vertical direction, the light receiving results of the plurality of pixels arranged two-dimensionally can be read out.

By capturing (reading out) the light receiving results of the returning light using the rolling shutter method for this type of image sensor 51, the light receiving image corresponding to the desired virtual opening shape extending in the row direction is acquired. Such control is disclosed in, for example, U.S. Patent No. 8237835.

FIG. 5 shows a diagram explaining the operation of the ophthalmic apparatus 1 according to the embodiments. FIG. 5 schematically represents an irradiated range IP of the slit-shaped illumination light irradiated on the fundus Ef and a virtual opening range OP on the light receiving surface SR of the image sensor 51.

For example, the controller 100 described below deflects the slit-shaped illumination light formed by the illumination optical system 20, using the optical scanner 30. Thereby, the irradiated range IP of the slit-shaped illumination light is sequentially moved in a direction (for example, the vertical direction) orthogonal to the slit direction (for example, the row direction, the horizontal direction) on the fundus Ef.

On the light receiving surface SR of the image sensor 51, by changing the pixels to be read out in units of lines by the controller 100 described below, the virtual opening range OP is set. The opening range OP is preferable to be the light receiving range IP' of the returning light of the illumination light on the light receiving surface SR or wider than the light receiving range IP'. The controller 100 described below performs the movement control of the opening range OP in synchronization with the movement control of the irradiated range IP of the illumination light. Thereby, without being affected by unnecessary scattered light, high quality images of the fundus Ef with strong contrast can be acquired using a simple configuration.

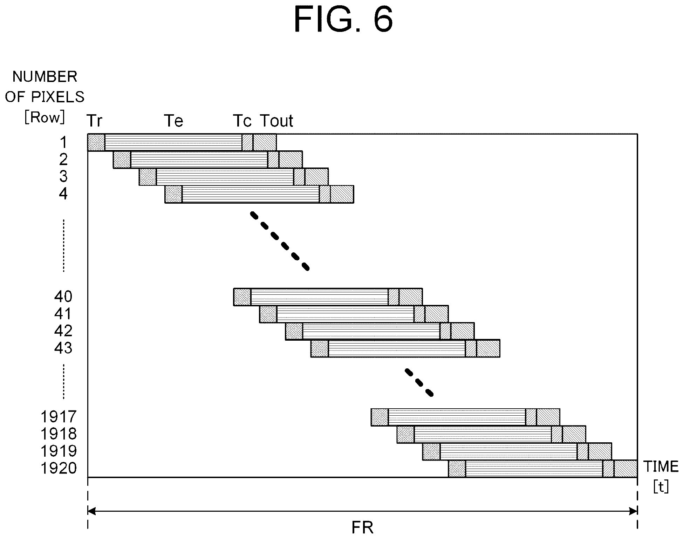

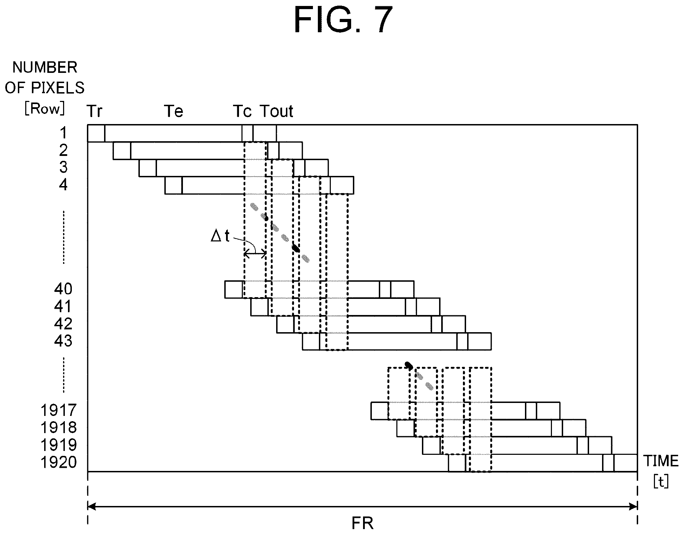

FIGs. 6 and 7 schematically show examples of the control timing of the rolling shutter method for the image sensor 51. FIG. 6 represents an example of the timing of the readout control for the image sensor 51. FIG. 7 represents the timing of the movement control for the irradiated range IP (the light receiving range IP') superimposed on the timing of the readout control in FIG. 6. In FIGs. 6 and 7, the horizontal axis represents the number of rows in the image sensor 51, and the vertical axis represents time.

In addition, in FIGs. 6 and 7, for convenience of explanation, it is assumed that the number of rows in the image sensor 51 is 1920. However, the configuration according to the embodiments is not limited to the number of rows. Further, in FIG. 7, for convenience of explanation, it is assumed that the slit width (width in the row direction) of the slit-shaped illumination light is 40 rows.

The readout control in the row direction includes the reset control, the exposure control, the charge transfer control, and the output control. The reset control is a control that initializes the amount of electrical charge accumulated in the pixels in the row direction. The exposure control is a control that illuminates light on the photodiode and accumulates the electrical charge corresponding to the amount of received light in the capacitor. The charge transfer control is a control that transfers the amount of the electrical charge accumulated in the pixel to the vertical signal line. The output control is a control that outputs the amount of the electrical charge accumulated in the plurality of vertical signal lines via the horizontal signal line. That is, as shown in FIG. 6, the readout time T for reading out the electrical charge accumulated in the pixels in the row direction is the sum of the time Tr required for the reset control, the time Te required for the exposure control (exposure time), the time Tc required for the charge transfer control, and the time Tout required for the output control.

In FIG. 6, by shifting the readout start timing (start timing of time Tc) in units of rows, the light receiving results (amount of electrical charge) accumulated in the pixels in the desired range in the image sensor 51 are acquired. For example, in case that the pixel range shown in FIG. 6 is for a single frame of the image, the frame rate FR is determined uniquely.

In this embodiment, the irradiated position of the illumination light on the fundus Ef, the illumination light having a slit width of a plurality of rows, is sequentially shifted in a direction corresponding to the column direction on the fundus Ef.

For example, as shown in FIG. 7, at each predetermined shift time Δt, the irradiated position of the illumination light on the fundus Ef is shifted in row units in the direction corresponding to the column direction. The shift time Δt is obtained by dividing the exposure time Te of the pixel in the image sensor 51 by the slit width (e.g., 40) of the illumination light (Δt = Te/40). Synchronized with this movement timing of this irradiated position, the readout start timing of each row of pixels is delayed and is started for each row in units of shift time Δt. This allows to acquired high quality images of the fundus Ef with strong contrast in a short time with a simple control.

In some embodiments, the image sensor 51 is configured using one or more line sensors.

### [Configuration of control system]

As shown in FIG. 2, the control system of the ophthalmic apparatus 1 is configured with a controller 100 as a center. It should be noted that at least a part of the configuration of the control system may be included in the ophthalmic apparatus 1.

### (Controller 100)

The controller 100 controls each part of the ophthalmic apparatus 1. The controller 100 includes a main controller 101 and a storage unit 102. The main controller 101 includes a processor and executes the control processing of each part of the ophthalmic apparatus 1 by executing processing according to the program(s) stored in the storage unit 102.

### (Main controller 101)

The main controller 101 performs control for the light source 10 and a movement mechanism 10D, control for the illumination optical system 20, control for the optical scanner 30, control for the imaging optical system 40, control for the imaging device 50, and control for the data processor 200.

The control for the light source 10 includes switching the light source on and off (or switching the wavelength region of the light), and changing the light amount of the light source.

The movement mechanism 10D changes at least one of the position of the light source 10 or the orientation of the light source 10 using a known mechanism. The main controller 101 can change at least one of a relative position of the light source 10 to the iris aperture 21 and the slit 22, or a relative orientation of the light source 10 to the iris aperture 21 and the slit 22.

The control for the illumination optical system 20 includes control for a movement mechanism 22D. The movement mechanism 22D moves the slit 22 in the optical axis direction of the illumination optical system 20. The main controller 101 controls the movement mechanism 22D in accordance with the state of the subject's eye E to arrange the slit 22 at the position corresponding to the state of the subject's eye E. Examples of the state of the subject's eye E includes a shape of the fundus Ef, a dioptric power, and an axial length. The dioptric power can be obtained from a known eye refractive power measurement apparatus as disclosed in Japanese Unexamined Patent Application No. 61- 293430 or Japanese Unexamined Patent Application Publication No. 2010-259495, for example. The axial length can be obtained from a known axial length measurement apparatus or a measurement value acquired by an optical coherence tomography.

For example, the storage unit 102 stores first control information. In the first control information, the positions of the slit 22 on the optical axis of the illumination optical system 20 are associated with the dioptric powers in advance. The main controller 101 specifies the position of the slit 22 corresponding to the dioptric power by referring to the first control information, and controls the movement mechanism 22D so as to arrange the slit 22 at the specified position.

Here, as the slit 22 moves, the light amount distribution of the light passing through the aperture formed in the slit 22 changes. In this case, as described above, the main controller 101 can control the movement mechanism 10D to change at least one of the position of the light source 10 or the orientation of the light source 10.

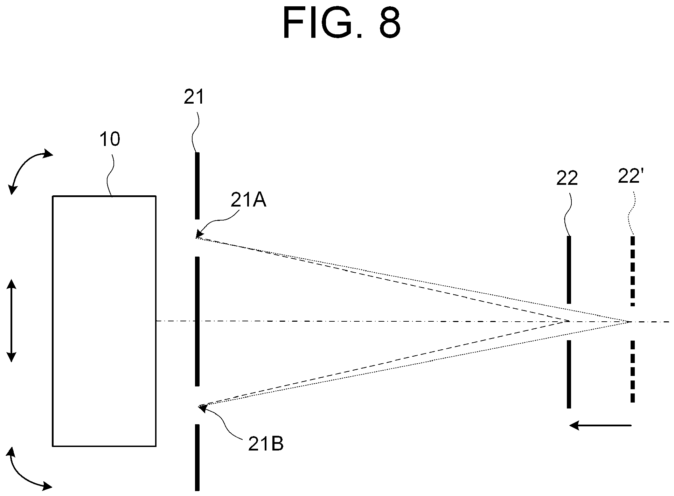

FIG. 8 shows a diagram describing the control content of the main controller 101 according to the embodiments. In Fig. 8, parts similar to those in Figs. 1 to 4 are denoted by the same reference symbols, and description thereof is omitted as appropriate.

As described above, the position of the slit 22 is moved from the position of the slit 22' before the movement according to the state of the subject's eye E. Thereby, the light amount distribution of the light passing through the aperture formed in the slit 22 changes.

In this case, the main controller 101 controls the movement mechanism 10D to change the relative position between the iris aperture 21 and the light source 10. By changing the relative position between the apertures 21A and 21 B, which are formed in the iris aperture 21, and the light source 10, the light amount distribution of the light passing through the apertures 21A and 21B is changed. Further, the light amount distribution of the light, which passes through the apertures 21A and 21B formed in the iris aperture 21, at the aperture formed in the slit 22 is changed.

The main controller 101 can control the movement mechanism 10D based on the dioptric power of the subject's eye E as the state of the subject's eye E and the position of the slit 22 after the movement (or movement direction and movement amount of the slit 22 with reference to a reference position).

For example, the storage unit 102 stores second control information. In the second control information, at least one of the positions or the orientations of the light source 10 are associated with the refractive powers and the positions of the slit 22 after the movement (or the movement directions and movement amounts of the slit 22 with reference to the reference position) in advance. The main controller 101 specifies at least one of the position of the light source 10 or the orientation of the light source 10 corresponding to the dioptric power or the position of the slit 22 after the movement by referring to the second control information, and controls the movement mechanism 10D so that the light source 10 is arranged at the specified position or in the specified orientation.

In FIG. 2, the control for the optical scanner 30 includes control of the scan range (scan start position and scan end position) and the scan speed.

The control for the imaging optical system 40 includes a control for a movement mechanism 47D. The movement mechanism 47D moves the focusing lens 47 in the optical axis direction of the imaging optical system 40. The main controller 101 can control the movement mechanism 47D based on an analysis result of the image acquired using the image sensor 51. Further, the main controller 101 can control the movement mechanism 47D based on a content of operation of the user using an operation unit 110 described below.

The control for the imaging device 50 includes a control for the image sensor 51 (rolling shutter control). The control for the image sensor 51 includes the reset control, the exposure control, the charge transfer control, and the output control. Further, time Tr required for the reset control, time (exposure time) Te required for the exposure control, time Tc required for the charge transfer control, and time Tout required for the output control, etc., can be changed.

Example of the control for the data processor 200 include various kinds of image processing and various kinds of analysis processing on the light receiving results acquired from the image sensor 51. Examples of the image processing include noise removal processing on the light receiving results, brightness correction processing for easily identifying a predetermined site depicted in the light receiving image based on the light receiving results. Examples of the analysis processing include a specifying processing of the in-focus state.

The data processor 200 can form the light receiving image corresponding to the arbitrary opening range based on the light receiving result(s) read out from the image sensor 51 using the rolling shutter method, under the control from the main controller 101 (controller 100). The data processor 200 can sequentially form light receiving light images corresponding to the opening ranges and can form an image of the subject's eye E from a plurality of formed light receiving images.

The data processor 200 includes a processor, and realizes the above functions by performing processing corresponding to the program(s) stored in the storage unit or the like.

In some embodiments, the light source 10 includes two or more light sources. In this case, each of the two or more light sources is provided corresponding to the two or more apertures formed in the iris aperture 21. The main controller 101 can change the at least one of a position of each light source or an orientation (orientation in the direction of maximum light amount distribution) of each light source, by controlling the movement mechanisms provided for each of the two or more light sources.

In some embodiments, at least one of the position of the optical element 24 or the orientation of the optical element 24 with respect to the aperture(s) formed in the iris aperture 21 can be changed. For example, the main controller 101 can change the at least one of the position of the optical element 24 or the orientation of the optical element 24 by controlling the movement mechanism that moves the optical element 24.

### (Storage unit 102)

The storage unit 102 stores various computer programs and data. The computer programs include an arithmetic program and a control program for controlling the ophthalmic apparatus 1.

### (Operation unit 110)

The operation unit 110 includes an operation device or an input device. The operation unit 110 includes buttons and switches (e.g., operation handle, operation knob, etc.) and operation devices (e.g., mouse, keyboard, etc.) provided in the ophthalmic apparatus 1. In addition, the operation unit 110 may include any operation device or any input device, such as a trackball, a control panel, a switch, a button, a dial, etc.

### (Display unit 120)

The display unit 120 displays the image of the subject's eye E generated by data processor 200. The display unit 120 is configured to include a display device such as a flat panel display such as an LCD (Liquid Crystal Display). In addition, the display unit 120 may include various types of display devices such as a touch panel and the like provided in the housing of the ophthalmic apparatus 1.

It should be noted that the operation unit 110 and the display unit 120 do not need to be configured to be separate devices. For example, a device like a touch panel, which has a display function integrated with an operation function, can be used. In this case, the operation unit 110 includes the touch panel and a computer program. The content for the operation unit 110 is fed to the controller 100 as electrical signals. Moreover, operations and inputs of information may be performed using a graphical user interface (GUI) displayed on the display unit 120 and the operation unit 110. In some embodiments, the functions of the display unit 120 and the operation unit 110 are realized a touch screen.

### (Other configurations)

In some embodiments, the ophthalmic apparatus 1 further includes a fixation projection system. For example, an optical path of the fixation projection system is coupled with the optical path of the imaging optical system 40 in the configuration of the optical system shown in FIG. 1. The fixation projection system can present internal fixation targets or external fixation targets to the subject's eye E. In case of presenting the internal fixation target to the subject's eye E, the fixation projection system includes an LCD that displays the internal fixation target under the control from the controller 100, and projects a fixation light flux output from the LCD onto the fundus Ef of the subject's eye E. The LCD is configured to be capable of changing the display position of the fixation target on the screen of the LCD. By changing the display position of the fixation target on the screen of the LCD, the projected position of the fixation target on the fundus of the subject's eye E can be changed. The display position of the fixation target on the LCD can be designated using the operation unit 110 by the user.

In some embodiments, the ophthalmic apparatus 1 includes an alignment system. In some embodiments, the alignment system includes an XY alignment system and a Z alignment system. The XY alignment system is used for position matching between the optical system of the apparatus and the subject's eye E in a direction intersecting the optical axis of the optical system of the apparatus (objective lens 46). The Z alignment system is used for position matching between the optical system of the apparatus and the subject's eye E in a direction of the optical axis of the ophthalmic apparatus 1 (objective lens 46).

For example, the XY alignment system projects a bright spot (bright spot in the infrared region or near-infrared region) onto subject's eye E. The data processor 200 acquires an anterior segment image of the subject's eye E on which the bright spot is projected, and obtains the displacement between the bright spot image drawn on the acquired anterior segment image and an alignment reference position. The controller 100 relatively moves the optical system of the apparatus and the subject's eye E in the direction intersecting the direction of the optical axis so as to cancel the obtained displacement, using the movement mechanism.

For example, the Z alignment system projects alignment light in infrared region or the near-infrared region from a position away from the optical axis of the optical system of the apparatus, and receives the alignment light reflected on the anterior segment of the subject's eye E. The data processor 200 specifies a distance to the subject's eye E with respect to the optical system of the apparatus, from the light receiving position of the alignment light that changes in accordance with the distance to the subject's eye E with respect to the optical system of the apparatus. The controller 100 relatively moves the optical system of the apparatus and the subject's eye E in the direction of the optical axis using the movement mechanism (not shown) so that the specified distance becomes a predetermined working distance.

In some embodiments, the function of the alignment system is realized by two or more anterior segment cameras arranged at positions away from the optical axis of the optical system of the apparatus. For example, as disclosed in Japanese Unexamined Patent Application Publication No. 2013-248376, the data processor 200 analyzes data processor segment images of subject's eye E substantially simultaneously acquired using the two or more anterior segment cameras, and specifies a three-dimensional position of the subject's eye E using known trigonometry. The controller 100 controls the movement mechanism (not shown) to relatively move the optical system of the apparatus and the subject's eye E three-dimensionally so that the optical axis of the optical system of the apparatus substantially coincides with an axis of the subject's eye E and the distance of the optical system of the apparatus with respect to the subject's eye E is a predetermined working distance.

The movement mechanism 22D is an example of the "first movement mechanism" according to the embodiments. The movement mechanism 10D is an example of the "second movement mechanism" according to the embodiments. A movement mechanism (not shown) that changes at least one of the position of the optical element 24 or the orientation of the optical element 24 is an example of the "third movement mechanism" according to the embodiments.

### [Operation]

Next, the operation of the ophthalmic apparatus 1 will be described.

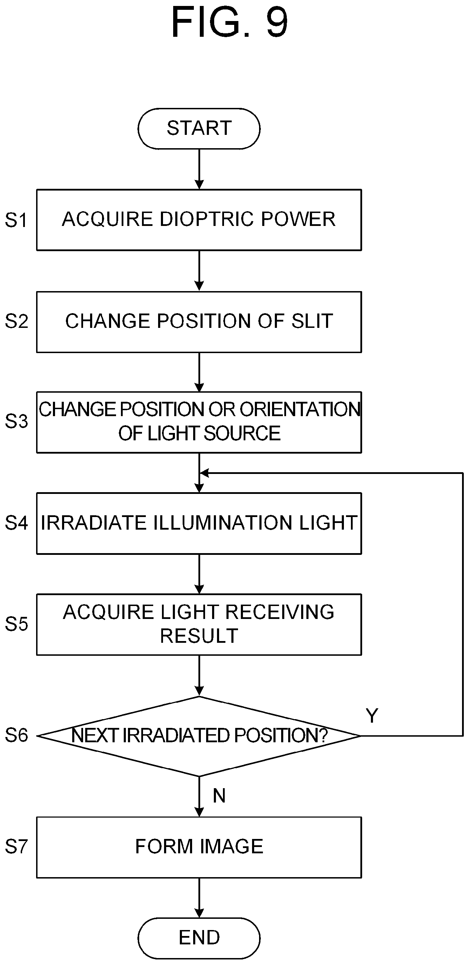

FIG. 9 shows a flow chart of an example of the operation of the ophthalmic apparatus 1 according to the embodiments. The storage unit 102 stores a computer program for realizing the processing shown in FIG. 9. The main controller 101 operates according to the computer program, and thereby the main controller 101 performs the processing shown in FIG. 9.

Here, it is assumed that the alignment of the optical system of the apparatus with respect the subject's eye E using the alignment system (not shown) is completed, and that the fixation target is projected onto the fundus of the subject's eye E to guide the subject's eye E to a desired fixation position using the fixation projection system (not shown).

### (S1: Acquire dioptric power)

First, the main controller 101 acquires the dioptric power of the subject's eye E from an external ophthalmic measurement apparatus or an electronic medical record.

### (S2: Change position of slit)

Next, the main controller 101 changes the position of the slit 22 on the optical axis of the illumination optical system 20 in accordance with the dioptric power of the subject's eye E acquired in step S1.

Specifically, the main controller 101 specifies the position of the slit 22 corresponding to the dioptric power by referring to the first control information stored in the storage unit 102, and controls the movement mechanism 22D so as to arrange the slit 22 at the specified position.

### (S3: Change position or orientation of light source)

Subsequently, the main controller 101 changes at least one of the position of the light source 10 or the orientation of the light source 10 in accordance with the new position of the slit 22 whose position on the optical axis has been changed in step S2.

Specifically, the main controller 101 specifies at least one of the position or the orientation of the light source 10 that correspond to the refractive power or the position of the slit 22 after the movement, by referring to the second control information stored in the storage unit 102. And then, the main controller 101 controls the movement mechanism 10D so that the light source 10 is arranged at the specified position or in the specified orientation.

### (S4: Irradiate illumination light)

Next, the main controller 101 controls the illumination optical system 20 to generate the slit-shaped illumination light, and to start the deflection control of the optical scanner 30 to start irradiating the illumination light onto a desired irradiated region on the fundus Ef. When the irradiation of the illumination light is started, the slit-shaped illumination light is sequentially irradiated within the desired irradiated range as described above.

### (S5: Acquire light receiving result)

The main controller 101 acquires the light receiving result(s) of the pixels in the opening range of the image sensor 51 corresponding to the irradiated range of the illumination light on the fundus Ef performed in step S4, as described above.

### (S6: Next irradiated position?)

The main controller 101 determines whether or not the next irradiated position is to be irradiated with the illumination light. The main controller 101 can determine whether or not the next irradiated position is to be irradiated with the illumination light, by determining whether or not the irradiated range of the illumination light that is moved sequentially has covered a predetermined imaging range of the fundus Ef.

When it is determined that the next irradiated position is to be irradiated with the illumination light (S6: Y), the operation of the ophthalmic apparatus 1 proceeds to step S4. When it is determined that the next irradiated position is not to be irradiated with the illumination light (S6: N), the operation of the ophthalmic apparatus 1 proceeds to step S7.

### (S7: Form image)

In step S6, when it is determined that the next irradiated position is not to be irradiated with the illumination light (S6: N), the main controller 101 controls the data processor 200 to form the image of the subject's eye E from the light receiving results acquired repeatedly while changing the irradiated range of the illumination light in step S5.

For example, the data processor 200 syntheses a plurality of light receiving results with different irradiated ranges (opening ranges on the light receiving surface SR of the image sensor 51) of the illumination light for the number of times repeating the process in steps S4 to S6, based on the order of the movement of the irradiated range. Thereby, the fundus image of the fundus Ef for one frame is formed.

In some embodiments, in step S4, the illumination light is irradiated on the irradiated range set so as to have an overlapping region with the adjacent irradiated range. Thereby in step S7, the fundus image for one frame is formed by synthesizing the overlapping regions so as to overlap with each other.

This terminates the operation of the ophthalmic apparatus 1 (END).

### <Second embodiment>

The configuration of the ophthalmic apparatus according to the embodiments is not limited to the configuration described in the first embodiment. For example, the ophthalmic apparatus according to a second embodiment can acquire a photographic image of the fundus Ef and an observation image of the fundus Ef. In this case, the light source 10 is used as a light source for photography and another light source different from light source 10 is used as a light source for observation. The photographic images are acquired using the same rolling shutter method as in the first embodiment. The observation images are also acquired by the same rolling shutter method as the photographic images.

Hereinafter, the ophthalmic apparatus according to the second embodiment will be described below mainly about the differences from the first embodiment.

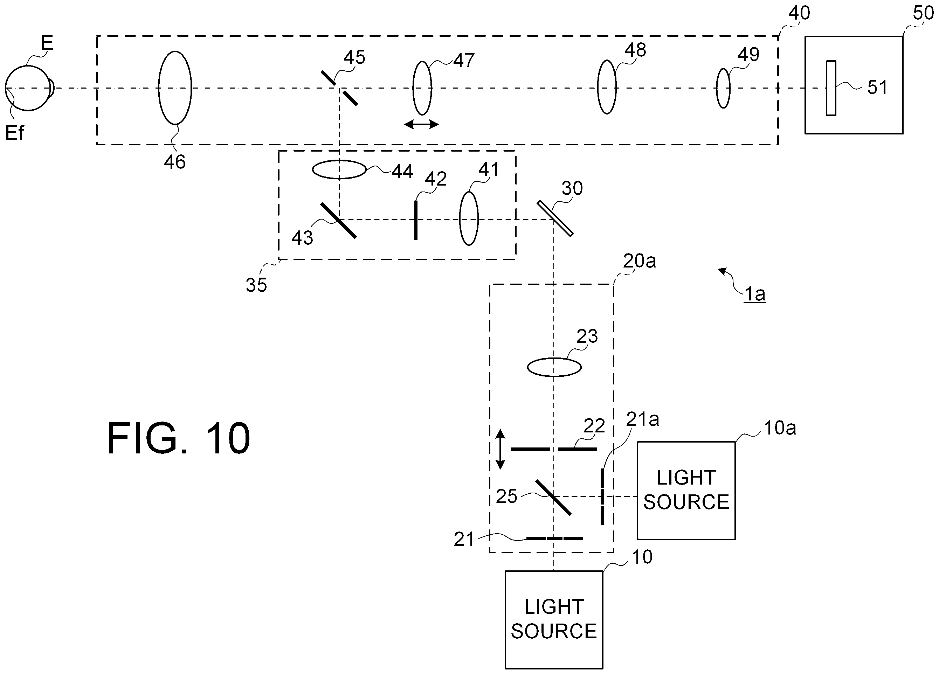

Fig. 10 shows an example of the configuration of the ophthalmic apparatus according to the second embodiment. In FIG. 10, like reference numerals designate like parts as in FIG. 1, and the same description may not be repeated.

The configuration of the ophthalmic apparatus 1a according to the second embodiment differs from that of the ophthalmic apparatus 1 according to the first embodiment in that an illumination optical system 20a is provided instead of the illumination optical system 20 and a light source 10a is added.

The configuration of the illumination optical system 20a differs from that of the illumination optical system 20 in that a dichroic mirror 25 as the optical path coupling member and an iris aperture 21a for observation are added. The dichroic mirror 25 is positioned between slit 22 and the iris aperture 21, and couples an optical path of light from the light source 10a with the optical path of the light from the light source 10. The iris aperture 21a is positioned between the light source 10a and the dichroic mirror 25.

The light source 10a includes an infrared light source that generates light in the infrared region. In some embodiments, the light source 10a includes a near-infrared light source that generates light in the near-infrared region. For example, the light source 10a generates light having a central wavelength in the wavelength range of 800 nm to 2500 nm. This type of light source 10a includes, for example, an LED, an LD, a halogen lamp, or a xenon lamp. The light source 10a is arranged at a position non-conjugate optically to each of the fundus Ef and the iris.

The iris aperture 21a (specifically, aperture(s)) can be arranged at a position substantially conjugate optically to the iris (pupil) of the subject's eye E. The iris aperture 21a has the same configuration as that of the iris aperture 21, and has one or more apertures.

The dichroic mirror 25 transmits the light from the light source 10, and reflects the light from the light source 10a toward the optical scanner 30.

In the present embodiment, the image sensor 51 is capable of detecting light in the visible and infrared regions. In this case, the controller according to the second embodiment performs the same control for the light source 10a as for the light source 10. For example, the controller turns on the light source 10 and turns off the light source 10a when photographing the fundus Ef, and turns on the light source 10a and turns off the light source 10 when observing the fundus Ef.

When the light source 10 is turned on, the visible light output from the light source 10 passes through the aperture(s) formed in the iris aperture 21, is transmitted through the dichroic mirror 25, passes through the aperture formed in slit 22, passed through the relay lens 23, and is guided to optical scanner 30. The light that has been guided to the optical scanner 30 illuminates the fundus Ef as in the first embodiment. Returning light of the illumination light from the fundus Ef is guided to the imaging device 50. That is, as in the first embodiment, the photographic image is acquired using the rolling shutter method.

When the light source 10a is turned on, the infrared light (or near-infrared light) output from the light source 10a passes through the aperture(s) formed in the iris aperture 21a, is reflected by the dichroic mirror 25, passes through the aperture formed in slit 22, passes through the relay lens 23, and is guided to optical scanner 30. The light that has been guided to the optical scanner 30 illuminates the fundus Ef as in the first embodiment. Returning light of the illumination light from the fundus Ef is guided to the imaging device 50. That is, the observation image is acquired using the same rolling shutter method as in the first embodiment. For example, the observation image is used for position matching of the optical system relative to the subject's eye E, and is used for observation of a photographed site, such as the fundus Ef.

The operation of the ophthalmic apparatus 1a according to the second embodiment is similar to the operation of the ophthalmic apparatus 1 according to the first embodiment. Thus, the detailed description of the operation will be omitted.

The light source 10 is an example of the "light source for photography" according to the embodiments. The light source 10a is an example of the "light source for observation" according to the embodiments. The dichroic mirror 25 is an example of the "optical path coupling member" according to the embodiments. The iris aperture 21a is an example of the "iris aperture for observation" according to the embodiments.

According to the second embodiment, the illumination intensity required for photographing (imaging) the fundus can be secured and high quality images of the subject's eye can be acquired while acquiring the observation image without being affected by the state of the subject's eye, with a simple configuration.

It should be noted that a case where the optical path of the light from the light source for observation is coupled to the optical path of the light from the light source for photography at a position on the side of the light source 10 relative to the slit 22, in the second embodiment. However, the configuration of the ophthalmic apparatus according to the embodiments is not limited thereto. The optical path of the light from the light source for observation can be coupled to the optical path of the light from the light source for photography at any position between the objective lens 46 and the iris aperture 21. In some embodiments, the light from the light source for observation is configured to enter the eye through the pupil without passing through the objective lens 46.

### <Third embodiment>

In the above embodiments, the aperture formed in the iris aperture 21 is described as having the shape shown in FIG .3. However, the shape of the aperture formed in the iris aperture according to the embodiments is not limited to the shape shown in FIG. 3.

Specifically, one or more apertures are formed in the iris aperture according to the embodiments so that a luminous flux cross section of the illumination light (illumination luminous flux cross section) and a luminous flux cross section of returning light from the subject's eye E (fundus Ef) (imaging luminous flux cross section) are separated on a reflective site in the path of the illumination light in the subject's eye E. The shape of the aperture formed in the iris aperture is not limited, as long as the illumination luminous flux cross section and the imaging luminous flux cross section are separated at the reflective site described above. Examples of the reflective site include a cornea (anterior surface of cornea, posterior surface of cornea), an anterior surface of lens, and a posterior surface of lens.

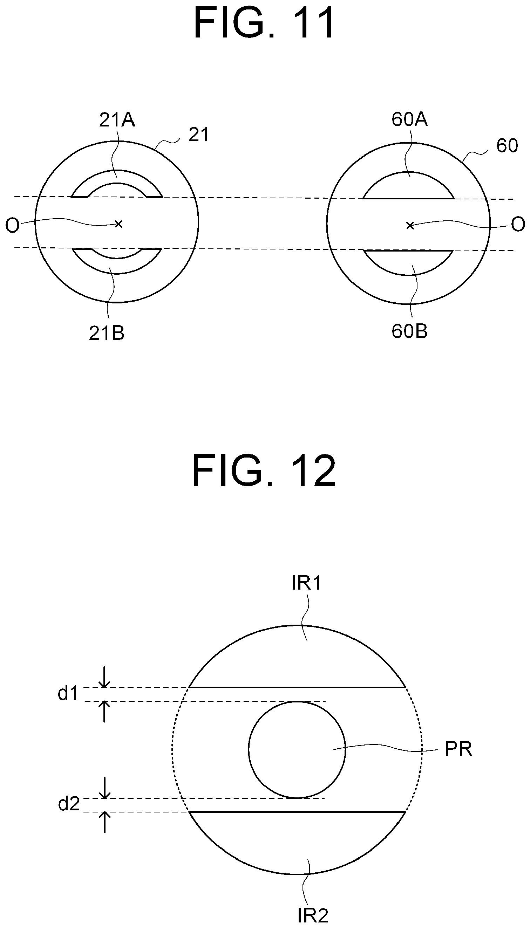

FIG. 11 shows an example of a configuration of the iris aperture according to the third embodiment. In FIG. 11, an iris aperture 60 according to the third embodiment is illustrated so that it can be contrasted with the iris aperture 21 shown in FIG. 3.

In the same way as the iris aperture 21, one or more apertures (in FIG. 11, apertures 60A and 60B) are formed in the iris aperture 60. In the same way as the apertures 21A and 21B, the apertures 60A and 60B are formed line-symmetrically with respect to a straight line extending through the position of the optical axis O in a direction corresponding to a longitudinal direction of the slit 22. The shape of the inner diameter of apertures 60A and 60B are defined by a straight line connecting two points on the inner diameter of apertures 21A and 21B so that the distance in the direction corresponding to the shorter direction of the slit 22 of the apertures 21A and 21B does not change.

In other words, each of the apertures 60A and 60B has a circular segment shape. The circular segment is the region bounded by the inferior arc of a circle or ellipse and the chord of this inferior arc. A direction of the chord of the circular segment shape is approximately parallel to a direction corresponding to the longitudinal direction of the aperture(s) formed in slit 22.

FIG. 12 schematically shows an example of the luminous flux cross section on the pupil of the subject's eye E in case of illuminating the subject's eye E using the iris aperture 60.

Light passing through the apertures 60A and 60B formed in the iris aperture 60 enters into the eye so as to form the luminous flux cross sections IR1 and IR2 on the pupil, for example. The luminous flux cross section IR1 is a luminous flux cross section of the light passing through the aperture 60A, for example. The luminous flux cross section IR2 is a luminous flux cross section of the light passing through the aperture 60B, for example.

The returning light (imaging light) that enters into the eye and is reflected on the fundus Ef forms the luminous flux cross section PR on the pupil, for example, and is guided to the imaging optical system 40.

In this case, the apertures 60A and 60B are formed so as to separate the luminous flux cross sections IR1 and IR2 of the illumination light and the luminous flux cross section PR of the imaging light.

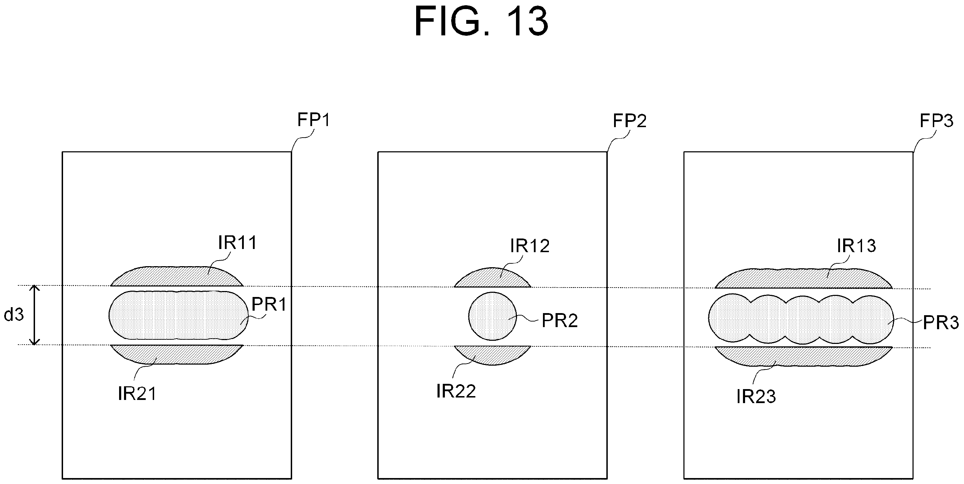

FIG. 13 schematically shows the illumination luminous flux cross section and the imaging luminous flux cross section at each part in the eye of the subject's eye E in case of illuminating the subject's eye E using the iris aperture 60. FIG. 13 schematically represents footprints FP1 to FP3 when the optical scanner 30 deflects with a predetermined deflection angle. The footprint FP1 represents the luminous flux cross section on the surface of the cornea. The footprint FP2 represents the luminous flux cross section on the anterior surface of lens (surface of the iris) (or surface of the photographic stop). The footprint FP3 represents the luminous flux cross section on the posterior surface of lens.

The anterior surface of lens (iris surface) (or surface of the photographic stop) is a position substantially conjugate optically to the iris aperture 60. Thereby, as shown in the footprint FP2, the same illumination luminous flux cross sections IR12 and IR22 and the imaging luminous flux cross section PR 2 as in FIG. 12 are formed. The respective shapes of the illumination luminous flux cross sections IR12 and IR22 are almost the same as the respective shapes of the apertures 60A and 60B formed in the iris aperture 60. The shape of the imaging luminous flux cross section PR2 is almost the same as the shape of the photographic stop (aperture formed in the perforated mirror 45). At the position substantially conjugate optically to the iris aperture 60, the illumination luminous flux cross section and the imaging luminous flux cross section are separated, as in the footprint FP2.

On the corneal surface, which is non-conjugate optically to the iris aperture 60, the illumination luminous flux cross sections IR11 and IR21 and the imaging luminous flux cross section PR1 spread in the direction corresponding to the longitudinal direction of the slit 22 (footprint FP1). Meanwhile, the relative relationship between the illumination luminous flux cross sections IR11 and IR21 and the imaging luminous flux cross section PR1 in the direction corresponding to the shorter direction of the slit 22 does not change.

In the same way, on the posterior surface of lens, which is non-conjugate optically to the iris aperture 60, the illumination luminous flux cross sections IR13 and IR23 and the imaging luminous flux cross section PR3 spread in the direction corresponding to the longitudinal direction of the slit 22 (footprint FP3). Meanwhile, the relative relationship between the illumination luminous flux cross sections IR13 and IR23 and the imaging luminous flux cross section PR3 in the direction corresponding to the shorter direction of the slit 22 does not change.

At the position, which is non-conjugate optically to the iris aperture 60, when the deflection angle of the illumination light is changed by the optical scanner 30, the positions of the illumination luminous flux cross section and the imaging luminous flux cross section move in the direction corresponding to the shorter direction of the slit 22. Even if the deflection angle changes, the relative relationship between the illumination luminous flux cross section and the imaging luminous flux cross section as shown in footprints FP1 and FP3 is maintained.

Therefore, the aperture 60A formed in the iris aperture 60 is required to be formed so that the distance d1 (distance in the direction corresponding to the shorter direction of the slit 22) between the lower end of the illumination luminous flux cross section (luminous flux cross section IR1) and the upper end of the imaging luminous flux cross section (luminous flux cross section PR) is greater than or equal to a predetermined first distance, as shown in FIG. 12. In the same way, the aperture 60B formed in the iris aperture 60 is required to be formed so that the distance d2 between the upper end of the illumination luminous flux cross section (luminous flux cross section IR2) and the lower end of the imaging luminous flux cross section (luminous flux cross section PR) is greater than or equal to a predetermined second distance, as shown in FIG. 12. Here, the first distance may be equal to the second distance. Further, the apertures 60A and 60B formed in the iris aperture 60 are required to be formed so that the distance d3 in the direction corresponding to the shorter direction of the slit 22 is greater than or equal to a predetermined third distance, as shown in FIG. 13.

That is, the shapes of the inner diameters of the apertures 60A and 60B does not contribute to the shapes of the illumination luminous flux cross section and the shape of the imaging luminous flux cross section.

As described above, the apertures 60A and 60B are formed in the iris aperture 60 so that the illumination luminous flux cross section and the imaging luminous flux cross section are separated at the cornea, the anterior surface of lens, and the posterior surface of lens of the subject's eye E. Thereby, in the same way as in the first embodiment and the second embodiment, without being affected by unnecessary scattered light, high quality images of the fundus Ef with strong contrast can be acquired using a simple configuration.

In particular, by shaping the apertures 60A and 60B as shown in FIG. 11, the light amount of the illumination light can be increased compared to the first and second embodiments, making it possible to acquire images with higher image quality.

### <Fourth embodiment>

The configuration of the ophthalmic apparatus according to the embodiments is not limited to the configurations described in the above embodiments. In a fourth embodiment, the optical system is configured according to Badal's principle. This allows to keep the size of the slit image at the fundus Ef constant, regardless the dioptric power of the subject's eye E.

Hereinafter, the ophthalmic apparatus according to the fourth embodiment will be described below mainly about the differences from the first embodiment.

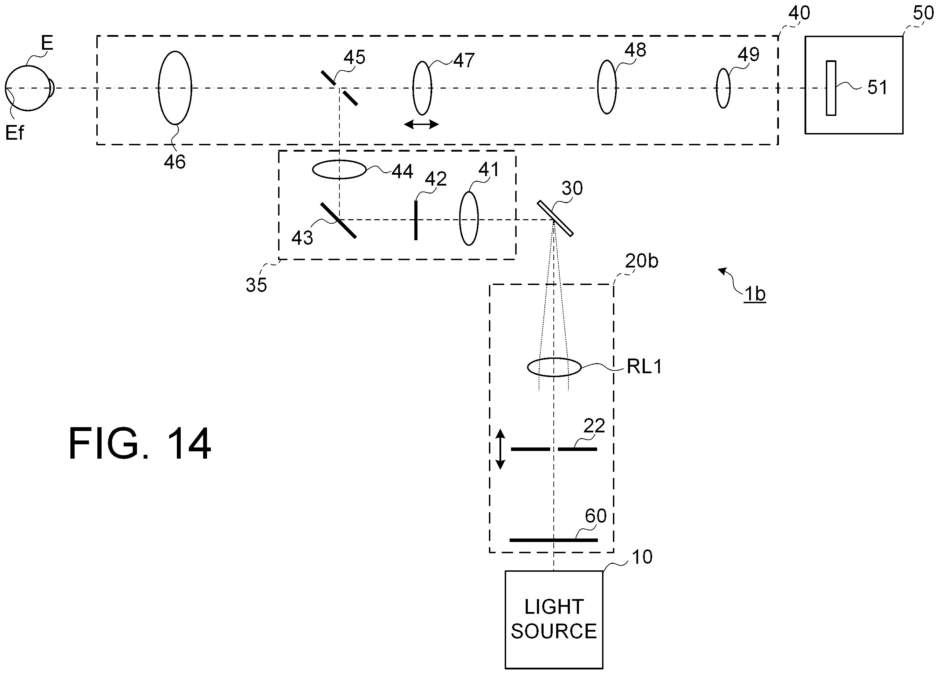

FIG. 14 shows an example of a configuration of the ophthalmic apparatus according to the fourth embodiment. In FIG. 14, like reference numerals designate like parts as in FIG. 1, and the same description may not be repeated.

The configuration of the ophthalmic apparatus 1b according to the fourth embodiment differs from that of the ophthalmic apparatus 1 according to the first embodiment in that an illumination optical system 20b is provided instead of the illumination optical system 20. It should be noted that in FIG. 14, the iris aperture 60 shown in FIG. 11 is provided instead of the iris aperture 21, but the configuration according to the fourth embodiment can be applied to the configuration with iris aperture 21.

The configuration of illumination optical system 20b differs from the configuration of the illumination optical system 20 in that a relay lens system RL1 is provided instead of the relay lens 23. That is, the relay lens system RL1 is arranged between the optical scanner 30 and the slit 22, in the same way as the relay lens 23. The relay lens system RL1, relay lenses 41 and 44, and the objective lens 46 constitute a Badal optical system.

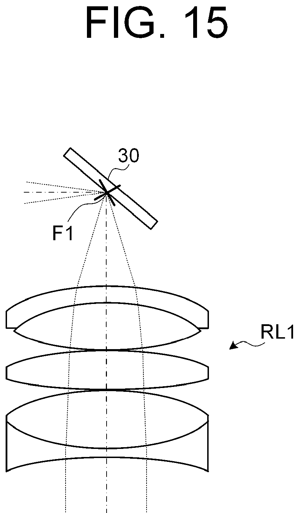

FIG. 15 shows an example of a configuration of the relay lens system RL1 according to the fourth embodiment. In FIG. 15, the relay lens system RL1 and the optical scanner 30 are illustrated for convenience of explanation. Further, in FIG. 15, the relay lens system RL1 is assumed to include three lenses.

In the same way as the relay lens 23, the relay lens system RL1 includes one or more lenses. A back focal position F1 of the relay lens system RL1 is arranged at a position substantially conjugate optically to the iris of the subject's eye E.

That is, the optical scanner 30, which is arranged at a position substantially conjugate optically to the iris of the subject's eye E as described above, is arranged at the back focal position F1 of the relay lens system RL1 or the vicinity of the back focal position F1. Therefore, even when the slit 22 is moved in the optical axis direction in accordance with the dioptric power of the subject's eye E, the size of the slit image (image formed by the light passing through the aperture formed in the slit 22) projected onto the fundus Ef does not change. This means that the projection magnification of the slit image onto the fundus Ef does not change even when the slit 22 moves in the optical axis direction.

The operation of the ophthalmic apparatus 1b according to the fourth embodiment is similar to the operation of the ophthalmic apparatus 1 according to the first embodiment. Thus, the detailed description of the operation will be omitted.

The relay lens system RL1 is an example of the "first relay lens system" according to the embodiments.

According to the fourth embodiment, by arranging the optical scanner 30 at the back focal position F1 of the relay lens system RL1 (or the vicinity of the back focal position F1), the Badal optical system is configured with the relay lens system RL1, the relay lenses 41 and 42, and the objective lens 46.

This allows to keep the projected angle of view (projection magnification) of the slit image with reference to the visual axis of the subject's eye E (longitudinal and shorter directions of the slit 22) constant, regardless the dioptric power of the subject's eye E. As a result, the size of the slit image does not change regardless of the dioptric power of the subject's eye E. This allows to keep the deflection operation speed of the optical scanner 30 constant, and to simplify the control of the optical scanner 30.

In addition, since the projected angle of view (projection magnification) of the slit image with reference to the visual axis of the subject's eye E is constant regardless of the dioptric power of the subject's eye E, the illumination intensity of the slit image at the fundus Ef can be kept constant regardless of the dioptric power of the subject's eye E.

Further, in case of acquiring images at a predetermined imaging angle of view in the ophthalmic apparatus, since the projection magnification is constant as described above, this eliminates the need for a margin of the length in the longitudinal length of the slit 22 provided to acquire a slit image of a predetermined size.

### <Fifth embodiment>

In the same way as in the second embodiment, the ophthalmic apparatus according to the fourth embodiment can acquire a photographic image of the fundus Ef and an observation image of the fundus Ef. In this case, the light source 10 is used as a light source for photography and another light source different from light source 10 is used as a light source for observation. The photographic images are acquired using the same rolling shutter method as in the first embodiment. The observation images are also acquired by the same rolling shutter method as the photographic images.

Hereinafter, the ophthalmic apparatus according to the fifth embodiment will be described below mainly about the differences from the fourth embodiment.

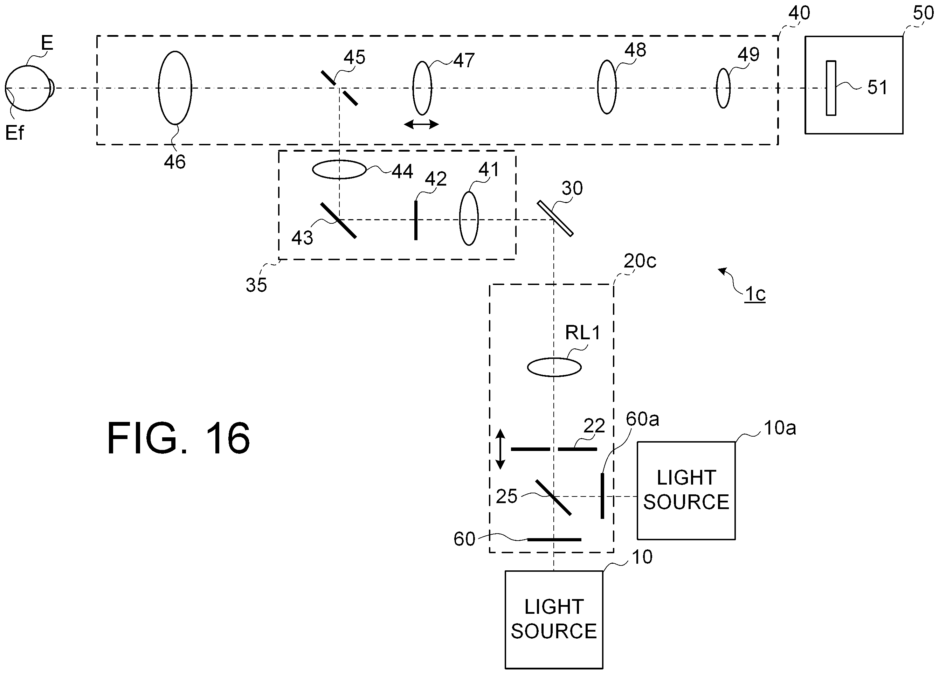

FIG. 16 shows an example of a configuration of the ophthalmic apparatus according to the fifth embodiment. In FIG. 16, like reference numerals designate like parts as in FIG. 14, and the same description may not be repeated.

The configuration of the ophthalmic apparatus 1c according to the fifth embodiment differs from that of the ophthalmic apparatus 1b according to the fourth embodiment in that an illumination optical system 20c is provided instead of the illumination optical system 20b and the light source 10a is added.

The configuration of the illumination optical system 20c differs from that of the illumination optical system 20b in that the dichroic mirror 25 as the optical path coupling member and an iris aperture 60a for observation are added. In the same way as in the second embodiment, the dichroic mirror 25 is positioned between slit 22 and the iris aperture 60, and couples an optical path of light from the light source 10a with the optical path of the light from the light source 10. The iris aperture 60a is positioned between the light source 10a and the dichroic mirror 25.

The light source 10a is the light source described in the second embodiment.

The iris aperture 60a (specifically, aperture(s)) can be arranged at a position substantially conjugate optically to the iris (pupil) of the subject's eye E. The iris aperture 60a has the same shape as that of the iris aperture 60, and has one or more apertures.

The dichroic mirror 25 is the dichroic mirror described in the second embodiment.

In the present embodiment, the image sensor 51 is capable of detecting light in the visible and infrared regions. In this case, the controller according to the fifth embodiment performs the same control for the light source 10a as for the light source 10. For example, the controller turns on the light source 10 and turns off the light source 10a when photographing the fundus Ef, and turns on the light source 10a and turns off the light source 10 when observing the fundus Ef.

When the light source 10 is turned on, the visible light output from the light source 10 passes through the aperture(s) formed in the iris aperture 60, is transmitted through the dichroic mirror 25, passes through the aperture formed in slit 22, passed through the relay lens system RL1, and is guided to optical scanner 30. The light that has been guided to the optical scanner 30 illuminates the fundus Ef as in the fourth embodiment. Returning light of the illumination light from the fundus Ef is guided to the imaging device 50. That is, as in the fourth embodiment, the photographic image is acquired using the rolling shutter method.

When the light source 10a is turned on, the infrared light (or near-infrared light) output from the light source 10a passes through the aperture(s) formed in the iris aperture 60a, is reflected by the dichroic mirror 25, passes through the aperture formed in slit 22, passes through the relay lens system RL1, and is guided to optical scanner 30. The light that has been guided to the optical scanner 30 illuminates the fundus Ef as in the fourth embodiment. Returning light of the illumination light from the fundus Ef is guided to the imaging device 50. That is, the observation image is acquired using the same rolling shutter method as in the fourth embodiment. For example, the observation image is used for position matching of the optical system relative to the subject's eye E, and is used for observation of a photographed site, such as the fundus Ef.

The operation of the ophthalmic apparatus 1c according to the fifth embodiment is similar to the operation of the ophthalmic apparatus 1b according to the fourth embodiment. Thus, the detailed description of the operation will be omitted.

The iris aperture 60a is an example of the "iris aperture for observation" according to the embodiments.

According to the fifth embodiment, the same effects as in the fourth embodiment can be achieved while acquiring the observation image.

It should be noted that the optical path of the light from the light source for observation is coupled to the optical path of the light from the light source for photography at a position on the side of the light source 10 relative to the slit 22, in the fifth embodiment. However, the configuration of the ophthalmic apparatus according to the embodiments is not limited thereto. The optical path of the light from the light source for observation can be coupled to the optical path of the light from the light source for photography at any position between the objective lens 46 and the iris aperture 21. In some embodiments, the light from the light source for observation is configured to enter the eye through the pupil without passing through the objective lens 46.

### <Sixth embodiment>

The configuration of the ophthalmic apparatus according to the embodiments is not limited to the configurations described in the fourth embodiment or the fifth embodiment. In the ophthalmic apparatus according to the sixth embodiment, a relay lens system is positioned between the slit 22 and the iris aperture 60 to increase the degree of freedom in optical design. In the following embodiment, the iris aperture 60 may be the iris aperture 21.

Hereinafter, the ophthalmic apparatus according to the sixth embodiment will be described below mainly about the differences from the fourth embodiment.

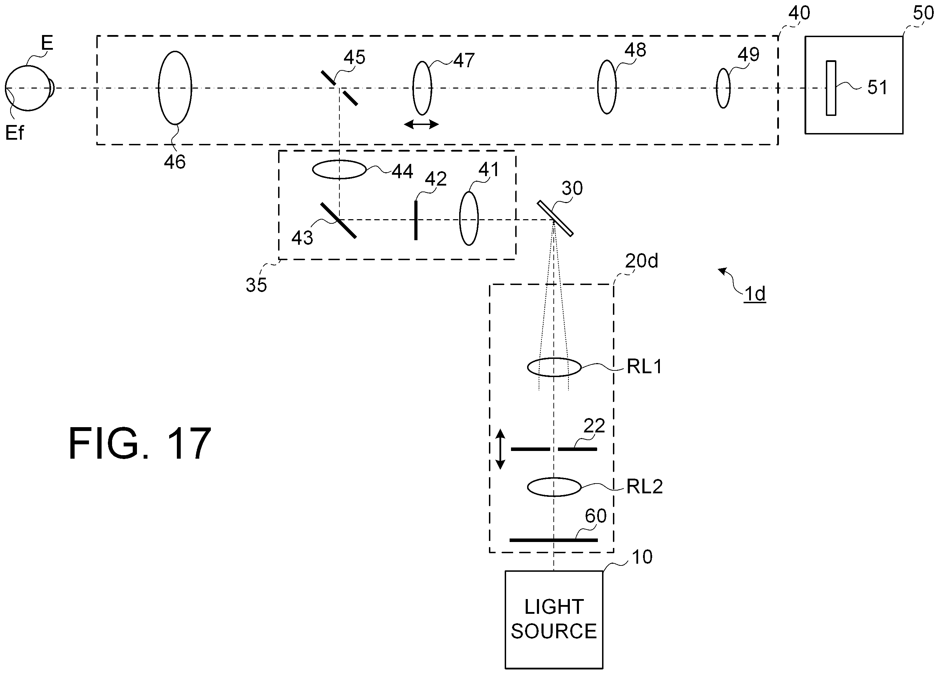

FIG. 17 shows an example of a configuration of the ophthalmic apparatus according to the sixth embodiment. In FIG. 17, components similar to those in FIG. 14 are given the same reference numerals. The description of such components is basically omitted.

The configuration of the ophthalmic apparatus 1d according to the sixth embodiment differs from that of the ophthalmic apparatus 1b according to the fourth embodiment in that an illumination optical system 20d is provided instead of the illumination optical system 20b.

The configuration of illumination optical system 20d differs from the configuration of the illumination optical system 20b in that a relay lens system RL2 is provided. That is, the relay lens system RL2 is arranged between the slit 22 and the iris aperture 60.

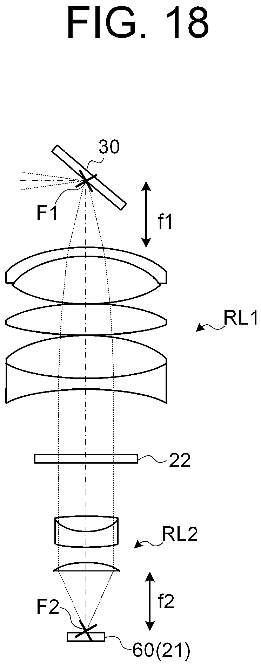

FIG. 18 shows an example of a configuration of the relay lens system RL2 according to the sixth embodiment. In FIG. 18, the iris aperture 60 (iris aperture 21), the relay lens system RL2, the slit 22, the relay lens system RL1, and the optical scanner 30 are illustrated for convenience of explanation. Further, in FIG. 18, the relay lens system RL2 is assumed to include two lenses.

In the same way as the relay lens system RL1, the relay lens system RL2 includes one or more lenses. The iris aperture 60 is arranged at a front focal position F2 of the relay lens system RL2 or the vicinity of the front focal position F2.

As described above, the iris aperture 60 is arranged at the front focal position F2 of the relay lens system RL2 or the vicinity of the front focal position F2. That is, the back focal position F1 of the relay lens system RL1 is the position substantially conjugate optically to the iris aperture 60, and the iris aperture 60 is arranged at the front focal position F2 of the relay lens system RL2. Therefore, the projection magnification from the iris aperture 60 to the optical scanner 30 (arranged at the back focal position F1) is determined by a focal distance f1 of the relay lens system RL1 and a focal distance f2 of the relay lens system RL2. In this case, the projection magnification is (f1/f2).

The ophthalmic apparatus according to the embodiments is required to form images of the iris aperture 60 with a predetermined size on the iris of the subject's eye E. When the projection magnification from the iris of the subject's eye E to the optical scanner 30 via the objective lens 46 is a known projection magnification, an image of the iris aperture 60 of a predetermined size should be projected on the optical scanner 30. In this case, the projection magnification from the iris aperture 60 to the optical scanner 30 is determined by the focal distance f1 of the relay lens system RL1 and the focal distance f2 of the relay lens system RL2. Therefore, by changing at least one of the focal distances f1 and f2, the image of the iris aperture 60 can be easily formed on the iris of the subject's eye E with a predetermined size. In some embodiments, while the focal distance f1 remains fixed, the focal distance f2 is changed alone.

The focal distance f1 is a composite focal distance of the relay lens system RL1. In some embodiments, the relay lens system RL1 includes a plurality of the lenses with different dioptric powers, and changes the focal distance f1 by changing at least one of the lenses constituting the relay lens system RL1. In some embodiments, at least one of the lenses constituting the relay lens system RL1 is a lens whose dioptric power can be changed. Examples of the lens whose dioptric power can be changed include a liquid crystal lens, a liquid lens, and an Alvarez lens. Even when the focal distance f1 is changed, the back focal position of the relay lens system RL1 is arranged at a position substantially conjugate optically to the iris (pupil conjugate position) of the subject's eye E.

The focal distance f2 is a composite focal distance of the relay lens system RL2. In some embodiments, the relay lens system RL2 includes a plurality of the lenses with different dioptric powers, and changes the focal distance f2 by changing at least one of the lenses constituting the relay lens system RL2. In some embodiments, at least one of the lenses constituting the relay lens system RL2 is a lens whose dioptric power can be changed. Even when the focal distance f2 is changed, the front focal position of the relay lens system RL2 is arranged at a position substantially conjugate optically to the iris (pupil conjugate position) of the subject's eye E.

In addition, for imaging the fundus Ef, it is desirable to use a light source that emits a high-intensity light. However, light sources available for general use (light sources that are mass-produced) are limited in the size of the emitting surface (luminous area, output luminous flux cross section size). Thereby, the image of the iris aperture 60 should be projected onto the optical scanner 30 with a projection magnification corresponding to the size of the emitting surface of the light source.

According to the present embodiment, by changing at least one of the focal distances f1 or f2, the projecting magnification from the iris aperture 60 to the optical scanner 30 can be changed. Thereby, the image of the iris aperture 60 with any size can be projected onto the optical scanner 30 with the desired size. This allows to project the image of the iris aperture 60 with a desired size onto the optical scanner 30 by simply changing at least one of the focal distances f1 or f2 even when the size of the emitting surface of the light source is different and to improve the degree of freedom in designing optical systems. In particular, this allows to fix the movement amount of the slit 22 in response to changes in the dioptric power of the subject's eye E (sensitivity of the movement of the slit 22 in response to changes in the dioptric power) by fixing the focal distance f1 and changing the focal distance f2 alone, and to further improve the degree of freedom in designing optical systems.

The operation of the ophthalmic apparatus 1d according to the sixth embodiment is similar to the operation of the ophthalmic apparatus 1b according to the fourth embodiment. Thus, the detailed description of the operation will be omitted.

The relay lens system RL2 is an example of the "second relay lens system" according to the embodiments.

According to the sixth embodiment, the effective diameter of one or more lenses constituting the relay lens system RL1 can be reduced.

The reason for this is that the slit 22, which is arranged at a position substantially conjugate optically to the fundus Ef of the subject's eye E, is arranged between the optical scanner 30 and the iris aperture 60. The slit 22 can be moved in the optical axis direction in accordance with the dioptric power of the subject's eye E. Here, the projection magnification from the iris aperture 60 to the optical scanner 30 is determined by the first distance, which is a distance between the optical scanner 30 and the relay lens system RL1, and the second distance, which is a distance between the iris aperture 60 and the relay lens system RL1. Thereby, when the first distance is shortened, the second distance should also be shortened. However, since it is necessary to maintain the conjugate relationship with the iris and the conjugate relationship with the fundus Ef while securing the space for movement of the slit 22 in the optical axis direction, the first distance becomes longer and the effective diameter of the relay lens system RL1 becomes larger. According to this embodiment, by providing the relay lens system RL2, the projection magnification can be adjusted using the relay lens system RL2 even if the first distance is shortened. This allows to shorten the first distance while maintaining the conjugate relationship with the iris and the conjugate relationship with the fundus Ef and securing the space for movement of the slit 22 in the optical axis direction, and to reduce the effective diameter of the one or more lenses constituting the relay lens system RL1.

Further, since the effective diameter of the one or more lenses constituting the relay lens system RL1 can be reduced, the length of the optical system from the optical scanner 30 to the light source 10 can be reduced.

### <Modification example of sixth embodiment>

In the sixth embodiment, at least one of the focal distance f1 or the focal distance f2 may be changeable in accordance with the type of light source 10. The ophthalmic apparatus according to a modification example of the sixth embodiment can change at least one of the focal distance f1 or the focal distance f2 in accordance with the size of the emitting surface (luminous area, output luminous flux cross section size) of the light source 10.

For example, the relay lens system RL1 changes the focal distance f1 in the same way as in the sixth embodiment, in accordance with the size of the emitting surface of the light source 10. For example, the relay lens system RL2 changes the focal distance f2 in the same way as in the sixth embodiment, in accordance with the size of the emitting surface of the light source 10.

In some embodiments, the main controller 101 changes the focal distance f1 by controlling the relay lens system RL1 (or a lens whose dioptric power can be changed) in accordance with the size of the emitting surface of the light source 10 designated using the operation unit 110. In some embodiments, the main controller 101 changes the focal distance f2 by controlling the relay lens system RL2 (or a lens whose dioptric power can be changed) in accordance with the size of the emitting surface of the light source 10 designated using the operation unit 110.

### <Seventh embodiment>

The ophthalmic apparatus according to the sixth embodiment or the modification example of the sixth embodiment can acquire the photographic image of the fundus Ef and the observation image of the fundus Ef, in the same way as in the second embodiment or the fifth embodiment. In this case, the light source 10 is used as a light source for photography and another light source different from light source 10 is used as a light source for observation. The photographic images are acquired using the same rolling shutter method as in the first embodiment. The observation images are also acquired by the same rolling shutter method as the photographic images.

Hereinafter, the ophthalmic apparatus according to the seventh embodiment will be described below mainly about the differences from the sixth embodiment.

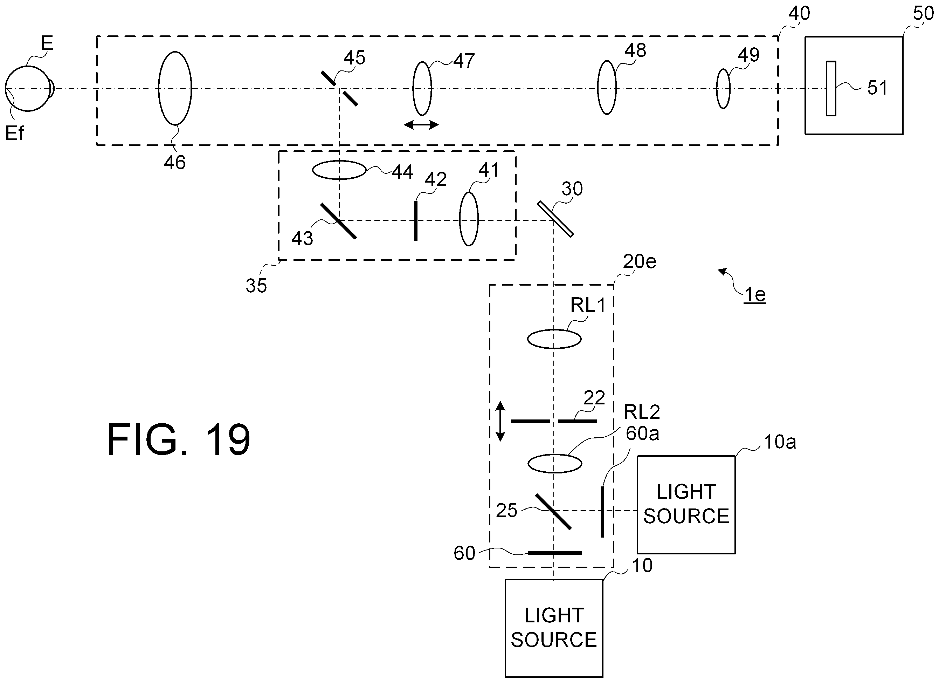

FIG. 19 shows an example of a configuration of the ophthalmic apparatus according to the seventh embodiment. In FIG. 19, like reference numerals designate like parts as in FIG. 16 or FIG. 17. The same description may not be repeated.

The configuration of the ophthalmic apparatus 1e according to the seventh embodiment differs from that of the ophthalmic apparatus 1d according to the sixth embodiment in that an illumination optical system 20e is provided instead of the illumination optical system 20d and the light source 10a is added.

The configuration of the illumination optical system 20e differs from that of the illumination optical system 20d in that the dichroic mirror 25 as the optical path coupling member and an iris aperture 60a for observation are added. In the same way as in the sixth embodiment, the dichroic mirror 25 is positioned between slit 22 and the iris aperture 60, and couples an optical path of light from the light source 10a with the optical path of the light from the light source 10. The iris aperture 60a is positioned between the light source 10a and the dichroic mirror 25.

The light source 10a is the light source described in the second embodiment (fifth embodiment).

The iris aperture 60a (specifically, aperture(s)) can be arranged at a position substantially conjugate optically to the iris (pupil) of the subject's eye E. The iris aperture 60a has the same shape as that of the iris aperture 60, and has one or more apertures.

The dichroic mirror 25 is the dichroic mirror described in the second embodiment (fifth embodiment).

In the present embodiment, the image sensor 51 is capable of detecting light in the visible and infrared regions. In this case, the controller according to the seventh embodiment performs the same control for the light source 10a as for the light source 10. For example, the controller turns on the light source 10 and turns off the light source 10a when photographing the fundus Ef, and turns on the light source 10a and turns off the light source 10 when observing the fundus Ef.

When the light source 10 is turned on, the visible light output from the light source 10 passes through the aperture(s) formed in the iris aperture 60, is transmitted through the dichroic mirror 25, passes through the aperture formed in slit 22, passed through the relay lens system RL1, and is guided to optical scanner 30. The light that has been guided to the optical scanner 30 illuminates the fundus Ef as in the fifth embodiment. Returning light of the illumination light from the fundus Ef is guided to the imaging device 50. That is, as in the sixth embodiment, the photographic image is acquired using the rolling shutter method.

When the light source 10a is turned on, the visible light output from the light source 10a passes through the aperture(s) formed in the iris aperture 21a, is reflected by the dichroic mirror 25, passes through the aperture formed in slit 22, passes through the relay lens system RL1, and is guided to optical scanner 30. The light that has been guided to the optical scanner 30 illuminates the fundus Ef as in the fifth embodiment. Returning light of the illumination light from the fundus Ef is guided to the imaging device 50. That is, the observation image is acquired using the same rolling shutter method as in the sixth embodiment. For example, the observation image is used for position matching of the optical system relative to the subject's eye E, and is used for observation of a photographed site, such as the fundus Ef.

The operation of the ophthalmic apparatus 1e according to the seventh embodiment is similar to the operation of the ophthalmic apparatus 1d according to the sixth embodiment. Thus, the detailed description of the operation will be omitted.

According to the seventh embodiment, the same effects as in the sixth embodiment can be achieved while acquiring the observation image.

It should be noted that the optical path of the light from the light source for observation is coupled to the optical path of the light from the light source for photography at a position on the side of the light source 10 relative to the slit 22, in the seventh embodiment. However, the configuration of the ophthalmic apparatus according to the embodiments is not limited thereto. The optical path of the light from the light source for observation can be coupled to the optical path of the light from the light source for photography at any position between the objective lens 46 and the iris aperture 21. In some embodiments, the light from the light source for observation is configured to enter the eye through the pupil without passing through the objective lens 46.

### [Actions and Effects]

The Actions and the effects of the ophthalmic apparatus and the method of controlling the ophthalmic apparatus according to the embodiments will be described.

An ophthalmic apparatus (1, 1a, 1b, 1c, 1d, 1e) according to some embodiments includes a light source (10), an illumination optical system (20), an optical scanner (30), an imaging optical system (40), and a controller (100, main controller 101). The illumination optical system is configured to generate slit-shaped illumination light using light from the light source. The optical scanner is configured to deflect the illumination light to guide the illumination light to a fundus (Ef) of a subject's eye (E). The imaging optical system is configured to guide returning light of the illumination light from the fundus to an image sensor (51). The controller is configured to control the image sensor using a rolling shutter method so as to acquire light receiving result of the returning light corresponding to an irradiated position of the illumination light on the fundus. The illumination optical system includes a slit (22) with a slit-shaped aperture capable of being arranged at a position substantially conjugate optically to the fundus, an iris aperture (21) arranged between the light source and the slit, and configured to be capable of being arranged at a position substantially conjugate optically to an iris of the subject's eye, and an optical element (24) arranged between the light source and the iris aperture, and configured to deflect the light from the light source.

According to such a configuration, the light passing through the iris aperture among the light from the light source can be deflected by the optical element and can be guided to the aperture formed in the slit. This allows to efficiently enter the light from the light source into the eye by the pupil division. Therefore, even when an inexpensive light source with a wide spread angle is used, the illumination intensity required for imaging the fundus can be secured and high quality images of the subject's eye can be acquired without being affected by the state of the subject's eye, with a simple configuration.

An ophthalmic apparatus (1, 1a, 1b, 1c, 1d, 1e) according to some embodiments includes a light source (10), an illumination optical system (20), a first movement mechanism (movement mechanism 22D), an optical scanner (30), an imaging optical system (40), and a controller (100, main controller 101). The illumination optical system includes a slit (22) with a slit-shaped aperture capable of being arranged at a position substantially conjugate optically to a fundus (Ef) of a subject's eye (E), and is configured to generate slit-shaped illumination light using light from the light source. The first movement mechanism is configured to move the slit in an optical axis direction of the illumination optical system. The optical scanner is configured to deflect the illumination light to guide the illumination light to the fundus. The imaging optical system is configured to guide returning light of the illumination light from the fundus to an image sensor (51). The controller is configured to control the image sensor using a rolling shutter method so as to acquire light receiving result of the returning light corresponding to an irradiated position of the illumination light on the fundus. The controller is configured to control the first movement mechanism based on a dioptric power of the subject's eye.

According to such a configuration, the position of the slit, which is positioned at a position substantially conjugate optically to the fundus of the subject's eye, is moved according to the dioptric power of the subject's eye. Thereby, the light from the light source light can be efficiently guided to the fundus of the subject's eye. This allows to efficiently enter the light from the light source into the eye by the pupil division. Therefore, even when an inexpensive light source with a wide spread angle is used, the illumination intensity required for imaging the fundus can be secured and high quality images of the subject's eye can be acquired without being affected by the state of the subject's eye, with a simple configuration.

The ophthalmic apparatus according to some embodiments further includes a second movement mechanism (10D) configured to change at least one of a position of the light source or an orientation of the light source. The controller is configured to control the second movement mechanism according to the position of the slit moved by the first movement mechanism.

According to such a configuration, even when the positional relationship between the light source and the slit is changed according to the dioptric power of the subject's eye, the light amount distribution in a direction connecting the light source and the aperture of the slit can be changed. Thereby, the fundus of the subject's eye can be illuminated with a desired illumination intensity, without being affected by the dioptric power of the subject's eye.

In the ophthalmic apparatus according to some embodiments, the illumination optical system includes an iris aperture (21, 60) arranged between the light source and the slit, and configured to be capable of being arranged at a position substantially conjugate optically to an iris of the subject's eye. The controller is configured to control the second movement mechanism so that light having passed through the iris aperture passes through the aperture.

According to such a configuration, even when the positional relationship between the light source and the slit is changed according to the dioptric power of the subject's eye, it can be adjusted so that the light passing through the iris aperture, through which the light from the light source is irradiated, passes through the aperture of the slit. Thereby, the fundus of the subject's eye can be illuminated with a desired illumination intensity, without being affected by the dioptric power of the subject's eye.

In the ophthalmic apparatus according to some embodiments, the illumination optical system includes a first relay lens system (relay lens system RL1) arranged between the optical scanner and the slit. A back focal position (F1) of the first relay lens system is a position substantially conjugate optically to the iris.

According to such an aspect, the optical system from the first relay lens system to the iris of the subject's eye can be configured according to the Badal's principle. Thereby, even when the slit is moved in the optical axis direction in accordance with the dioptric power of the subject's eye, the size of the slit image projected onto the fundus of the subject's eye does not change, regardless of the dioptric power of the subject's eye. This means that the projection magnification of the slit image onto the fundus does not change even when the slit moves in the optical axis direction. As a result, regardless of the dioptric power of the subject's eye, this allows to keep the deflection operation speed of the optical scanner constant, and to simplify the control of the optical scanner. In addition, since the projected angle of view (projection magnification) of the slit image with reference to the visual axis of the subject' eye is constant regardless of the dioptric power of the subject's eye, the illumination intensity of the slit image on the fundus can be kept constant regardless of the dioptric power of the subject's eye. Further, in case of acquiring images at a predetermined imaging angle of view in the ophthalmic apparatus, since the projection magnification is constant, this eliminates the need for a margin longitudinal length of the slit provided to acquire a slit image of a predetermined size.

In some embodiments, the optical scanner is arranged at the back focal position or the vicinity of the back focal position.

According to such a configuration, regardless of the dioptric power of the subject's eye, this allows to keep the deflection operation speed of the optical scanner constant while reducing the size of the optical system, and to simplify the control of the optical scanner.

The ophthalmic apparatus according to some embodiments further includes a light source (light source 10a) for observation, an optical path coupling member (dichroic mirror 25) arranged between the first relay lens system and the iris aperture, and configured to couple an optical path of light output from the light source and an optical path of light output from the light source for observation, and an iris aperture (iris aperture 60a) for observation arranged between the light source for observation source and the optical path coupling member.

According to such a configuration, the illumination intensity required for photographing the fundus can be secured and high quality images of the subject's eye can be acquired while acquiring the observation image without being affected by the state of the subject's eye, with a simple configuration.

The ophthalmic apparatus according to some embodiments further includes a second relay lens system (relay lens system RL2) arranged between the slit and the iris aperture. The iris aperture is arranged at a front focal position of the second relay lens system or the vicinity of the front focal position of the second relay lens system.

According to such a configuration, by changing at least one of the focal distance of the first relay lens system or the focal distance of the second relay lens system, the projection magnification from the iris aperture to the optical scanner can be changed. Thereby, the image of the iris aperture with any size can be projected onto the optical scanner with a desired size. This allows to project the image of the iris aperture with the desired size onto the optical scanner even when the size of the emitting surface of the light source is different, and to improve the degree of freedom in designing optical systems.

In the ophthalmic apparatus according to some embodiments, at least one of a dioptric power of the first lens or a dioptric power of the second lens can be changed.

According to such a configuration, the image of the iris aperture with any size can be projected onto the optical scanner with the desired size. This allows to project the image of the iris aperture with the desired size onto the optical scanner even when the size of the emitting surface of the light source is different, and to improve the degree of freedom in designing optical systems.

In some embodiments, at least one of the dioptric power of the first lens or the dioptric power of the second lens can be changed according to a size of a light emitting surface of the light source.

According to such a configuration, the high quality image of the fundus can be acquired at low cost, without being affected by the size of the emitting surface of the light source.

The ophthalmic apparatus according to some embodiments further includes a light source (10a) for observation, an optical path coupling member (dichroic mirror 25) arranged between the second relay lens system and the iris aperture, and configured to couple an optical path of light output from the light source and an optical path of light output from the light source for observation; and an iris aperture (iris aperture 60a) for observation arranged between the light source for observation and the optical path coupling member.

According to such a configuration, the illumination intensity required for photographing the fundus can be secured and high quality images of the subject's eye can be acquired while acquiring the observation image without being affected by the state of the subject's eye, with a simple configuration.

In the ophthalmic apparatus according to some embodiments, the iris aperture has one or more apertures that the illumination light passes through so that luminous flux cross section of the illumination light and luminous flux cross section of returning light from the subject's eye are separated on a cornea of the subject's eye, an anterior surface of lens of the subject's eye, and a posterior surface of lens of the subject's eye.

According to such a configuration, by pupil-dividing the illumination light incident on the subject's eye and the returning light from the subject's eye with a high degree of accuracy, the illumination required for imaging the fundus can be secured and high quality image of the subject's eye can be acquired, with a simple configuration, without being affected by the state of the subject's eye.

In the ophthalmic apparatus according to some embodiments, the iris aperture has two or more apertures. The two or more apertures are formed in linear symmetry to a straight line, the straight line passing through an optical axis of the illumination optical system and extending in a direction corresponding to a longitudinal direction of the aperture formed in the slit.

According to such a configuration, the illumination light incident on the subject's eye from the fundus different directions and the returning light from the subject's eye can be perform pupil division with a high degree of accuracy.

In the ophthalmic apparatus according to some embodiments, the aperture has a circular segment shape, and a direction of a chord of the circular segment shape is approximately parallel to a direction corresponding to the longitudinal direction of the aperture formed in the slit.

According to such a configuration, the light amount of illumination light can be increased and the high quality images of the fundus with stronger contrast can be acquired, with a simple configuration.

In the ophthalmic apparatus according to some embodiments, the illumination optical system includes an optical element (24) arranged between the light source and the iris aperture, and configured to deflect light from the light source.

According to such a configuration, the light passing through the iris aperture among the light from the light source can be deflected by the optical element and can be guided to the aperture formed in the slit. This allows to efficiently enter the light from the light source into the eye by the pupil division. Therefore, even when an inexpensive light source with a wide spread angle is used, the illumination intensity required for imaging the fundus can be secured with a simple configuration.

In the ophthalmic apparatus according to some embodiments, the optical element is configured to deflect the light from the light source so that a light amount distribution in a direction connecting the iris aperture and the aperture is maximized.

According to such a configuration, even when an inexpensive light source is used, the fundus can be illuminated with a desired illumination intensity with a simple configuration.

The ophthalmic apparatus according to some embodiments further includes a third movement mechanism configured to change at least one of a position of the optical element or an orientation of the optical element. The controller is configured to control the third movement mechanism.

According to such a configuration, at least one of the position of the optical element or the orientation of the optical element is changed. Thereby, the light amount distribution in the direction connecting the iris aperture and the aperture of the slit can be adjusted. Thereby, even when the positional relationship between the light source and the iris aperture, the light amount distribution in the direction connecting the iris aperture and the aperture can be adjusted.

In the ophthalmic apparatus according to some embodiments, the optical element includes a prism, a microlens array, or a Fresnel lens.

This allows to efficiently enter the light from the light source into the eye by the pupil division. Therefore, the illumination intensity required for imaging the fundus can be secured with a simple configuration.

A method of controlling an ophthalmic apparatus (1, 1a, 1b, 1c, 1d, 1e) according to some embodiments is a method of controlling the ophthalmic apparatus including a light source (10), an illumination optical system (20), a first movement mechanism (movement mechanism 22D), an optical scanner (30), an imaging optical system (40), and a controller (100, main controller 101). The illumination optical system includes a slit (22) with a slit-shaped aperture capable of being arranged at a position substantially conjugate optically to a fundus (Ef) of a subject's eye (E), and is configured to generate slit-shaped illumination light using light from the light source. The first movement mechanism is configured to move the slit in an optical axis direction of the illumination optical system. The optical scanner is configured to deflect the illumination light to guide the illumination light to the fundus. The imaging optical system is configured to guide returning light of the illumination light from the fundus to an image sensor (51). The controller is configured to control the image sensor using a rolling shutter method so as to acquire light receiving result of the returning light corresponding to an irradiated position of the illumination light on the fundus. The method of controlling the ophthalmic apparatus includes an acquisition step of acquiring a dioptric power of the subject's eye; and a first control step of controlling the first movement mechanism based on the dioptric power acquired in the acquisition step.

According to such a method, the position of the slit, which is positioned at a position substantially conjugate optically to the fundus of the subject's eye, is moved according to the dioptric power of the subject's eye. Thereby, the light from the light source light can be efficiently guided to the fundus of the subject's eye. This allows to efficiently enter the light from the light source into the eye by the pupil division. Therefore, even when an inexpensive light source with a wide spread angle is used, the illumination intensity required for imaging the fundus can be secured and high quality images of the subject's eye can be acquired without being affected by the state of the subject's eye, with a simple configuration.

In the method of controlling the ophthalmic apparatus according to some embodiments, the ophthalmic apparatus includes a second movement mechanism (movement mechanism 10D) configured to change at least one of a position of the light source or an orientation of the light source. The method further includes a second control step of controlling the second movement mechanism according to the position of the slit moved by the first movement mechanism.

According to such a method, even when the positional relationship between the light source and the slit is changed according to the dioptric power of the subject's eye, the light amount distribution in a direction connecting the light source and the aperture of the slit can be changed. Thereby, the fundus of the subject's eye can be illuminated with a desired illumination intensity, without being affected by the dioptric power of the subject's eye.

In the method of controlling the ophthalmic apparatus according to some embodiments, the illumination optical system includes an iris aperture (21, 60) arranged between the light source and the slit, and configured to be capable of being arranged at a position substantially conjugate optically to an iris of the subject's eye. The second control step is performed to control the second movement mechanism so that light having passed through the iris aperture passes through the aperture.

According to such a method, even when the positional relationship between the light source and the slit is changed according to the dioptric power of the subject's eye, it can be adjusted so that the light passing through the iris aperture, through which the light from the light source is irradiated, passes through the aperture of the slit. Thereby, the fundus of the subject's eye can be illuminated with a desired illumination intensity, without being affected by the dioptric power of the subject's eye.

In the method of controlling the ophthalmic apparatus according to some embodiments, the illumination optical system includes an optical element (24) arranged between the light source and the iris aperture, and configured to deflect light from the light source. The ophthalmic apparatus includes a third movement mechanism configured to change at least one of a position of the optical element or an orientation of the optical element. The method of controlling the ophthalmic apparatus further includes a third control step of controlling the third movement mechanism.

According to such a method, at least one of the position of the optical element or the orientation of the optical element is changed. Thereby, the light amount distribution in the direction connecting the iris aperture and the aperture of the slit can be adjusted. Thereby, even when the positional relationship between the light source and the iris aperture, the light amount distribution in the direction connecting the iris aperture and the aperture can be adjusted.

In the method of controlling the ophthalmic apparatus according to some embodiments, the optical element includes a prism, a microlens array, or a Fresnel lens.

According to such a method, this allows to efficiently enter the light from the light source into the eye by the pupil division. Therefore, the illumination intensity required for imaging the fundus can be secured with a simple configuration.

The above-described some embodiments or the modification examples thereof are merely examples for carrying out the present invention. Those who intend to implement the present invention can apply any modification, omission, addition, or the like within the scope of the gist of the present invention.

In the above embodiments, the ophthalmic apparatus may have arbitrary functions adaptable in the field of ophthalmology. Examples of such functions include an axial length measurement function, a tonometry function, an optical coherence tomography (OCT) function, an ultrasonic inspection, and the like. It should be noted that the axial length measurement function is realized by the OCT, etc. Further, the axial length measurement function may be used to measure the axial length of the subject's eye by projecting light onto the subject's eye and detecting the returning light from the fundus while adjusting the position of the optical system in the Z direction (front-back direction) relative to the subject's eye. The intraocular pressure measurement function is realized by the tonometer, etc. The OCT function is realized by the OCT apparatus, etc. The ultrasonic inspection function is realized by the ultrasonic diagnosis apparatus, etc. Further, the present invention can also be applied to an apparatus (multifunctional apparatus) having two or more of such functions.

In some embodiments, a program for causing a computer to execute the method of controlling the ophthalmic apparatus described above is provided. Such a program can be stored in any non-transitory computer-readable recording medium. Examples of the recording medium include a semiconductor memory, an optical disk, a magneto-optical disk (CD-ROM, DVD-RAM, DVD-ROM, MO, etc.), a magnetic storage medium (hard disk, floppy (registered trade mark) disk, ZIP, etc.), and the like. The computer program may be transmitted and received through a network such as the Internet, LAN, etc.

The configurations described in the first embodiment to the seventh embodiment and the modification example of the sixth embodiment can be combined as desired.

### [Explanation of symbols]

1, 1a, 1b, 1c, 1d, 1e Ophthalmic apparatus
10, 10a Light source
20, 20a, 20b, 20c, 20d, 20e Illumination optical system
21, 21a, 60, 60a Iris aperture
22 Slit
23, 41, 44, 48 Relay lens
25 Dichroic mirror
30 Optical scanner
35 Projection optical system
40 Imaging optical system
42 Black point plate
43 Reflective mirror
45 Perforated mirror
46 Objective lens
47 Focusing lens
49 Imaging lens
50 Imaging device
51 Image sensor
E Subject's eye
Ef Fundus
RL1, RL2 Relay lens system

## Claims

1. A method of controlling an ophthalmic apparatus, the ophthalmic apparatus comprising:
a light source;
an illumination optical system including a slit with a slit-shaped aperture capable of being arranged at a position substantially conjugate optically to a fundus of a subject's eye, and configured to generate slit-shaped illumination light using light from the light source;
a first movement mechanism configured to move the slit in an optical axis direction of the illumination optical system;
an optical scanner configured to deflect the illumination light to guide the illumination light to the fundus;
an imaging optical system configured to guide returning light of the illumination light from the fundus to an image sensor; and
a controller configured to control the image sensor using a rolling shutter method so as to acquire light receiving result of the returning light corresponding to an irradiated position of the illumination light on the fundus,
the method comprising:
an acquisition step of acquiring a dioptric power of the subject's eye; and
a first control step of controlling the first movement mechanism based on the dioptric power acquired in the acquisition step.

2. The method of controlling the ophthalmic apparatus of claim 1, wherein
the ophthalmic apparatus includes a second movement mechanism configured to change at least one of a position of the light source or an orientation of the light source, and
the method further includes a second control step of controlling the second movement mechanism according to the position of the slit moved by the first movement mechanism.

3. The method of controlling the ophthalmic apparatus of claim 2, wherein
the illumination optical system includes an iris aperture arranged between the light source and the slit, and configured to be capable of being arranged at a position substantially conjugate optically to an iris of the subject's eye, and
the second control step is performed to control the second movement mechanism so that light having passed through the iris aperture passes through the aperture.

4. The method of controlling the ophthalmic apparatus of claim 3, wherein
the illumination optical system includes an optical element arranged between the light source and the iris aperture, and configured to deflect light from the light source,
the ophthalmic apparatus includes a third movement mechanism configured to change at least one of a position of the optical element or an orientation of the optical element, and
the method further includes a third control step of controlling the third movement mechanism.

5. The method of controlling the ophthalmic apparatus of claim 4, wherein
the optical element includes a prism, a microlens array, or a Fresnel lens.
